(19) Europäisches Patentamt
European Patent Office
Office européen des brevets

(11) Numéro de publication: **0 076 713**
**B1**

# (12) FASCICULE DE BREVET EUROPEEN

(45) Date de publication du fascicule du brevet: **08.01.86**

(51) Int. Cl.⁴: **C 07 D 209/08**, A 61 K 31/40

(21) Numéro de dépôt: **82401660.4**

(22) Date de dépôt: **13.09.82**

## (54) Nouveaux dérivés de l'aminométhyl 1H-indole 4-méthanol et leurs sels, leur procédé et les intermédiaires de préparation, leur application à titre de médicaments, les compositions les renfermant.

(30) Priorité: **17.09.81 FR 8117560**

(43) Date de publication de la demande: **13.04.83 Bulletin 83/15**

(45) Mention de la délivrance du brevet: **08.01.86 Bulletin 86/2**

(84) Etats contractants désignés: **AT BE CH DE FR GB IT LI LU NL SE**

(56) Documents cités:
**FR - A - 1 532 210**
**FR - A - 2 332 010**

**HELVETICA CHIMICA ACTA, vol. 51, no. 7, 31 octobre 1968, pages 1616-1628, F. TROXLER et al.: "Synthesen von Indolen mit (2-Aminoäthyl)-, (2-Aminopropyl)- oder Alkanolamin-Seitenketten am Sechsring"**
**HOUBEN-WEYL: "Methoden der organischen Chemie", Band XI/1, 1957, Georg Thieme Verlag, Stuttgart, DE.**

(73) Titulaire: **ROUSSEL-UCLAF, 35, boulevard des Invalides, F-75007 Paris (FR)**

(72) Inventeur: **Guillaume, Jacques, 15, Avenue du Belvédère Apt. 1904, F-93310 Le Pré-Saint-Gervais (FR)**
Inventeur: **Nedelec, Lucien, 45, boulevard de l'Ouest, F-93340 Le Raincy (FR)**
Inventeur: **Plassard, Guy, 6, rue Clément Marot, F-91600 Savigny-sur-Orge (FR)**
Inventeur: **Brown, Neil Leslie, 12, rue Jaucourt, F-75012 Paris (FR)**

(74) Mandataire: **Bourgouin, André et al, ROUSSEL-UCLAF 111, route de Noisy Boîte postale no 9, F-93230 Romainville (FR)**

**EP 0 076 713 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'aminométhyl-1H-indole-4-méthanol ainsi que leurs sels, le procédé de préparation, l'application à titre de médicaments de ces nouveaux dérivés, les compositions les renfermant et leurs intermédiaires de préparation.

Le brevet français N° 1532210 divulgue de manière très générale une famille de dérivés de l'aminométhyle méthanol substitués, au niveau du méthanol, par divers systèmes cycliques, dont l'indole, et doués de propriétés β-bloquantes.

L'invention a pour objet les dérivés de l'aminométhyl-1H-indole-4-méthanol, ainsi que leurs sels d'addition avec les acides minéraux ou organiques, caractérisé en ce qu'ils répondent à la formule générale I:

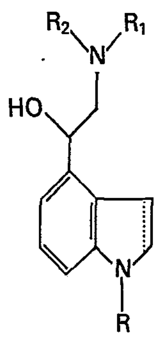

(I)

dans laquelle R représente un autome d'hydrogène ou un radical alcoyle renfermant de 1 à 6 atomes de carbone ou phényl alcoyle renfermant de 7 à 10 atomes de carbone et $R_1$ et $R_2$ identiques ou différents, représentent un atome d'hydrogène, un radical alcoyle linéaire ou ramifié renfermant de 1 à 8 atomes de carbone, éventuellement substitué, sauf dans le cas du radical méthyle, par un ou plusieurs atomes d'halogène ou par un ou plusieurs radicaux hydroxy, phényle ou phénoxy eux-mêmes éventuellement substitués par un ou plusieurs atomes d'halogène ou par un ou plusieurs radicaux hydroxy, méthyle, méthoxy ou acétamido, $R_1$ et $R_2$ peuvent également représenter un radical cycloalcoyle renfermant de 3 à 7 atomes de carbone, un radical cycloalcoylalcoyle renfermant de 4 à 7 atomes de carbone ou un radical allyle ou proparyle et le pointillé représente la présence éventuelle d'une liaison carbone-carbone.

Dans la formule générale I et dans ce qui suit, le terme radical alcoyle renfermant de 1 à 6 atomes de carbone, désigne de préférence, un radical méthyle, éthyle ou n-propyle, le terme radical phénylalcoyle désigne, de préférence, un radical benzyle ou phénéthyle; le terme radical alcoyle, linéaire ou ramifié renfermant de 1 à 8 atomes de carbone, désigne par exemple, un radical méthyle, éthyle, n-propyle, isopropyle, n-butyle, isobutyle ou pentyle, et de préférence, un radical méthyle, éthyle, propyle, isopropyle ou isobutyle; le radical alcoyle lorsqu'il est substitué comporte de préférence un seul substituant; le terme radical cycloalcoyle renfermant de 3 à 7 atomes de carbone, désigne, par exemple, un radical cyclopropyle mais est de préférence un radical cyclobutyle ou cyclopentyle; le terme radical cycloalcoylalcoyle renfermant de 4 à 7 atomes de carbone désigne, de préférence, un radical cyclopropylméthyle.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, propionique, benzoïque, maléique, fumarique, succinique, tartrique, citrique, oxalique, glyoxylique, aspartique, alcanesulfoniques, tels que les acides méthane et éthanesulfoniques, arylsulfoniques, tels que les acides benzène et paratoluènesulfoniques et arylcarboxyliques.

Parmi les produits objet de l'invention, on peut citer notamment les dérivés répondant à la formule (I) ci-dessus, ainsi que leurs sels d'addition avec les acides minéraux ou organiques, caractérisés en ce que, dans ladite formule (I), R représente un atome d'hydrogène, un radical alcoyle renfermant de 1 à 3 atomes de carbone, ou un radical benzyle, $R_1$ et $R_2$ représentent un atome d'hydrogène ou un radical alcoyle linéaire ou ramifié renfermant de 1 à 8 atomes de carbone, éventuellement substitué.

Parmi ceux-ci, on peut citer plus particulièrement les dérivés caractérisés en ce que, dans ladite formule I, R représente un atome d'hydrogène, $R_1$ et $R_2$ représentent un atome d'hydrogène ou un radical alcoyle, linéaire ou ramifié renfermant de 1 à 8 atomes de carbone, éventuellement substitué par un radical phényle ou phénoxy, eux-mêmes éventuellement substitués par un atome d'halogène, et le pointillé représente une liaison carbone-carbone, et tout particulièrement:

1'α-(aminométhyl)-1H-indole-4-méthanol,

1'α-[[(1-méthyléthyl)amino]méthyl]-1H-indole-4-méthanol,

1'α-(méthylamino)méthyl-1H-indole-4-méthanol,

1'α-[[(1-propyl)amino]méthyl]-1H-indole-4-méthanol,

ainsi que leurs sels d'addition avec les acides minéraux ou organiques.

On peut citer également les autres dérivés mentionnés ci-après dans la patie expérimentale.

L'invention a également pour objet un procédé de préparation des dérivés, tels que définis par la formule I ci-dessus, ainsi que de leurs sels, caractérisé en ce que l'on fait réagir un produit de formule II:

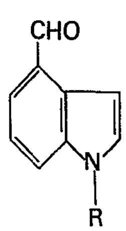

(II)

dans laquelle R a la signification déjà indiquée, avec le cyanure de triméthyl silyle, pour obtenir un produit de formule III:

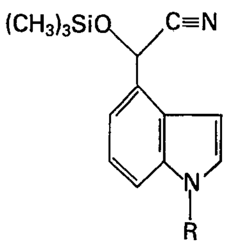

(III)

dans laquelle R a la signification déjà indiquée, que l'on réduit, pour obtenir un produit de formule $I_A$:

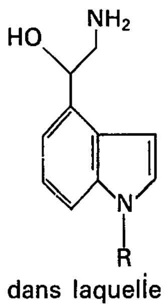

($I_A$)

dans laquelle R a la signification déjà indiquée, que:
soit l'on isole et, si désiré, salifie,
soit l'on fait réagir ledit produit de formule $I_A$ avec un dérivé carbonylé de formule IV:

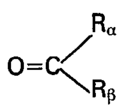

(IV)

dans laquelle Rα et Rβ sont tels que: 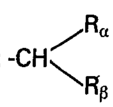

a la signification de $R_1$ déjà indiqué, à l'exception d'un atome d'hydrogène et du radical méthyle, en présence d'un agent de réduction, pour obtenir un produit de formule $I_B$:

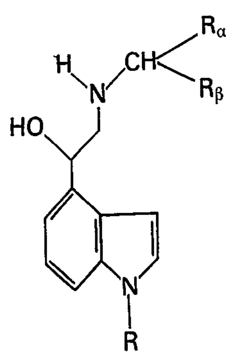

($I_B$)

ou $I'_B$:

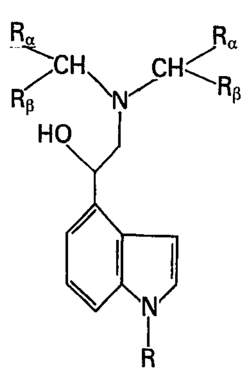

($I'_B$)

dans laquelle R, Rα et Rβ ont la signification déjà indiquée, produit de formule $I_B$ ou $I'_B$ que l'on isole et, si désiré, salifie, ou produit de formule $I_B$ que l'on soumet à l'action d'un halogénure de formule V:

$X–R'_2$ (V)

dans laquelle X représente un atome de chlore, de brome ou d'iode, et $R'_2$ a la signification de $R_2$ déjà indiquée, à l'exception d'un atome d'hydrogène, pour obtenir un produit de formule $I_C$:

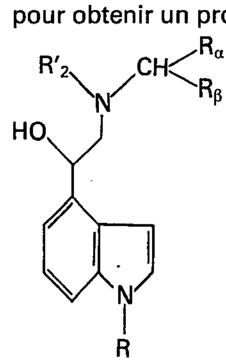

($I_C$)

dans laquelle R, Rα, Rβ et $R'_2$ ont la signification déjà indiquée, que l'on isole et, si désiré, salifie,
soit l'on fait réagir ledit produit de formule $I_A$ avec un dérivé carbonylé de formule $IV_a$:

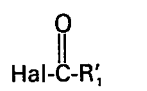

($IV_a$)

dans laquel Hal représente un atome de chlore, de brome ou d'iode et

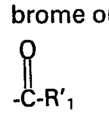

est tel que:

$-CH_2-R'_1$

a la signification de $R_1$ déjà indiquée, à l'exception d'un atome d'hydrogène et du radical méthyle, puis soumet le dérivé obtenu à l'action d'un agent de réduction, pour obtenir un produit de formule $I_D$:

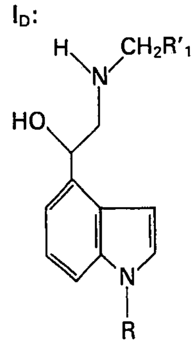

($I_D$)

dans laquelle R et $R'_1$ ont la signification déjà indiquée, produit de formule $I_D$ que ou bien l'on isole et, si désiré, salifie, ou bien l'on soumet à l'action d'un halogénure de formule V:

$X–R'_2$ (V)

dans laquelle X et $R'_2$ ont la signification déjà indiquée, pour obtenir un produit de formule $I_E$:

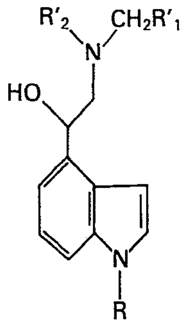

($I_E$)

dans laquelle R, $R'_1$ et $R'_2$ ont la signification déjà indiquée, que l'on isole et, si désiré, salifie, soit l'on soumet ledit produit de formule $I_A$ à une méthylation, pour obtenir un produit de formule $I_F$:

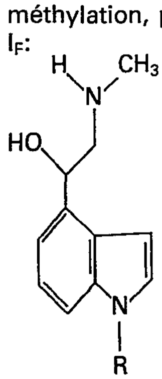

($I_F$)

dans laquelle R a la signification déjà indiquée, que ou bien l'on isole et, si désiré, salifie, ou bien l'on fait réagir avec un halogénure de formule V défini ci-dessus pour obtenir un produit de formule $I_G$:

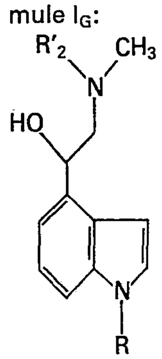

($I_G$)

dans laquelle R et $R'_2$ ont la signification déjà indiquée, que l'on isole et, si désiré, salifie, et, si désiré, soumet lesdits produits de formules $I_A$, $I_B$, $I'_B$, $I_C$, $I_D$, $I_E$, $I_F$ et $I_G$, à l'action d'un agent de réduction du noyau indole, pour obtenir un produit de formule I':

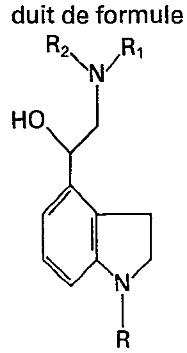

(I')

dans laquelle R, $R_1$ et $R_2$ ont la signification déjà indiquée, que l'on isole et, si désiré, salifie.

La réaction du produit de formule II avec le cyanure de triméthylsilyle est réalisée avantageusement en présence d'une quantité catalytique d'un acide de Lewis, tel que le chlorure d'aluminium ou de préférence l'iodure de zinc.

La réduction du produit de formule III est réalisée, de préférence, à l'aide d'hydrure d'aluminium lithium au sein d'un solvant tel que le tétrahydrofuranne.

La réaction du produit de formule $I_A$ avec le dérivé carbonylé de formule IV est avantageusement réalisée dans un solvant tel que l'acétonitrile ou de préférence un alcanol tel que l'éthanol ou particulièrement le méthanol.

La réaction peut, si désiré, être réalisée en présence d'une base telle que la triéthylamine ou la pyridine.

L'agent de réduction conduisant au produit de formule $I_B$ ou $I'_B$ peut être un borohydrure ou un cyanoborohydrure alcalin, tels que ceux de potassium ou de sodium; c'est de préférence l'hydrogène gazeux en présence d'un catalyseur à base de palladium.

L'halogénure de formule V est, de préférence, un iodure. On le fait avantageusement réagir avec les produits de formules $I_B$, $I_D$ ou $I_F$ en présence d'un agent fixateur d'hydracides comme, par exemple, un carbonate alcalin tel que le carbonate de potassium, un bicarbonate alcalin tel que le bicarbonate de sodium, un hydroxyde alcalin tel que la soude ou la potasse, une amine tertiaire, telle qu'une trialcoylamine ou la pyridine, ou encore un alcoolate alcalin tel que l'éthylate de sodium.

Le dérivé carbonylé de formule $IV_a$ est, de préférence, un chlorure. On opère notamment dans le tétrahydrofuranne en présence d'une base.

L'agent de réduction conduisant au produit de formule $I_D$ est, de préférence, un hydrure métallique, par exemple, l'hydrure d'aluminium lithium.

La méthylation du produit de formule $I_A$ est réalisée de préférence par réaction dudit produit de formule $I_A$ avec un halogénoformiate d'alcoyle, de préférence le chloroformiate d'éthyl, puis réduction du carbamate obtenu, de préférence à l'aide d'hydrure d'aluminium-lithium au reflux du tétrahydrofuranne. La réaction du produit de formule $I_A$ avec l'halogénoformiate d'alcoyle est avantageusement réalisée en présence d'une base telle que le carbonate de sodium ou la triéthylamine. La méthylation peut également être réalisée, par exemple, par action du formol et du méthanol, suivie d'une réduction par le borohydrure de sodium.

L'agent de réduction du noyau indole est de préférence, le complexe triméthylamine-borane dans un solvant tel que le dioxane en présence d'acide chlrohydrique.

L'invention a aussi pour objet une variante du procédé de préparation ci-dessus décrit, caractérisée en ce que l'on fait réagir le 4-chloro-indole avec un halogénure de formule $V_a$:

Hal–R' ($V_a$)

dans laquelle Hal représente un atome de chlore, de brome ou d'iode, et R' a la signification de R à l'exception d'hydrogène, pour obtenir un produit de formule VI:

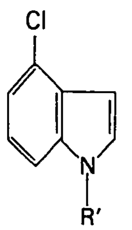

(VI)

dans laquelle R' a la signification déjà indiquée, que l'on transforme en magnésien correspondant, sur lequel on fait réagir un agent d'acétylation pour obtenir un produit de formule VII:

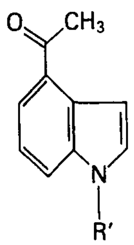

(VII)

dans laquelle R'a la signification déjà indiquée, dont on prépare l'oxime en α de la cétone, puis réduit le produit obtenu, pour obtenir un produit de formule $I'_A$:

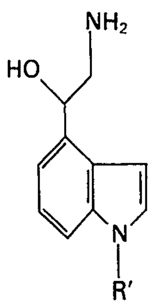

($I'_A$)

dans laquelle R' a la signification déjà indiquée, que soit l'on isole et, si désiré, salifie, soit poursuit le procédé comme indiqué ci-dessus pour le produit de formule $I_A$.

L'halogénure de formule Va est de préférence en chlorure. On le fait avantageusement réagir avec le 4-chloro indole en présence d'un agent fixateur d'acides tel qu'un carbonate ou un hydroxyde alcalin, le carbonate de sodium ou l'hydroxyde de potassium par exemple, et de préférence l'hydroxyde de sodium.

La formation du magnésien du produit de formule VI est réalisée de préférence, en faisant réagir le produit de formule VI avec le magnésium, dans un solvant approprié tel que le tétrahydrofurane, en présence d'une petite quantité de dibromoéthane.

L'agent d'acétylation peut être, par exemple, un halogénure d'acétyle, un anhydride mixte tel que l'anhydride mixte pivaloylacétique, le thiolacétate de 2-pyridyle, mais de préférence l'anhydride acétique.

On opère avantageusement à froid entre −20 et −40°C, et de préférence, l'on ajoute le magnésien au réactif d'acétylation dans un solvant tel que le tétrahydrofurane.

L'oximation du produit de formule VII est réalisée de préférence en milieu basique; on utilise avantageusement le mélange nitrite de ter-butyleterbutylate de potassium ter-butanol, dans un solvant tel que le tétrahydrofurane. On opère aux environs de 0°C, de préférence entre 0 et 5°C.

L'oxime étant peu stable, sa réduction a lieu de préférence in situ, aussitôt après la formation. Cette réduction peut être réalisée en deux opérations, par réduction de la fonction oxime, par exemple par hydrogénation catalytique en utilisant de préférence le palladium comme catalyseur, puis réduction de la fonction cétone, de préférence à l'aide de borohydrure de sodium. On opère de préférence en une seule opération, par réduction à l'aide d'hydrure d'aluminium-lithium par exemple.

L'invention a également pour objet une variante du procédé ci-dessus décrit, pour la préparation des produits de formule I, dans laquelle $R_1$ représente un radical alcoyle, linéaire ou ramifié, renfermant de 2 à 8 atomes de carbone, substitué par un ou plusieurs radicaux hydroxy dont au moins un hydroxy sur le carbone en position 2 et, en outre, éventuellement substitué par un ou plusieurs atomes d'halogène ou par un ou plusieurs radicaux aryle ou aryloxy éventuellement substitués sur le noyau aryle, et $R_2$ représente un atome d'hydrogène ou un radical alcoyle renfermant de 1 à 8 atomes de carbone, éventuellement substitué par un radical aryle, variante caractérisée en ce que l'on traite un produit de formule $I_H$:

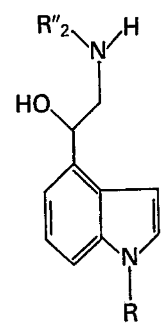

($I_H$)

dans laquelle R est défini comme ci-dessus et $R''_2$ représente un radical alcoyle renfermant de 1 à 8 atomes de carbone, éventuellement substitué par un radical aryle correspondant à un produit de formule $I_D$ dans laquelle $-CH_2-R'_1$ a la signification de $R''_2$ indiquée précédemment, par un réactif de formule VIII:

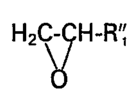

(VIII)

dans laquelle $R''_1$ est tel que $-CH_2-CH-R''_1$ a la va-
OH

leur de $R_1$ indiquée ci-dessus, pour obtenir un produit de formule $I_I$:

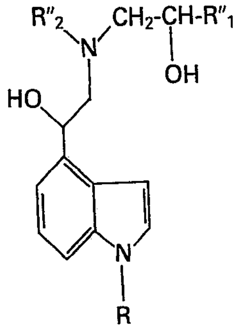

$(I_I)$

que, le cas échéant, l'on soumet à l'action d'un agent de réduction, pour obtenir un composé de formule $I_J$:

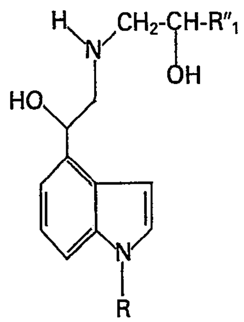

$(I_J)$

que soit l'on isole et, si désiré, salifie,

soit l'on soumet, comme les produits de formule $I_B$, $I_D$ ou $I_F$, à l'action d'un halogénure de formule V telle que définie précédemment, pour obtenir un produit de formule $I_K$:

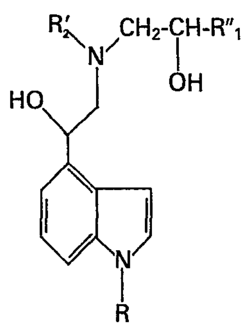

$(I_K)$

que l'on isole et, si désiré, salifie et, si désiré, soumet les produits de formule $I_I$, $I_J$ ou $I_K$ à l'action d'un agent de réduction du noyau indole, pour obtenir le produit réduit correspondant que l'on isole et, si désiré, salifie.

La réaction du produit de formule $I_H$ avec le réactif de formule VI est effectuée, de préférence, au sein d'un alcool comme le méthanol ou l'éthanol, ou d'un hydrocarbure aromatique.

L'agent de réduction du produit de formule $I_I$ est de préférence l'hydrogène en présence d'un catalyseur à base de palladium. Dans ce produit de formule $I_I$, $R''_2$ représente notamment un radical benzyle.

L'halogénure de formule V est l'agent de réduction du noyau indole sont, de préférence, ceux mentionnés précédemment.

Les dérivés de formule I présentent un caractère basique. On peut avantageusement préparer les sels d'addition des dérivés de formule I, en faisant réagir, en proportions sensiblement stoechiométriques, un acide minéral ou organique avec ledit dérivé de formule I. Les sels peuvent être préparés sans isoler les bases correspondantes.

Les dérivés, objet de la présente invention, possèdent de très intéressantes propriétés pharmacologiques; ils sont doués notamment de remarquables propriétés anti-hypertensives et hypotensives, ainsi que, pour certains, de propriétés antiulcéreuses.

Ces propriétés sont illustrées plus loin dans la partie expérimentale.

Ces propriétés justifient l'utilisation des dérivés de l'aminométhyl 1H-indole-4-méthanol ainsi que de leurs sels pharmaceutiquement acceptables, à titre de médicaments.

La présente demande a ainsi également pour objet l'application à titre de médicaments des dérivés de l'aminométhyl 1H-indole-4-méthanol, tels que définis par la formule I, ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés de l'aminométhyl 1H-indole-4-méthanol répondant à la formule I dans laquelle R représente un atome d'hydrogène, un radical alcoyle renfermant de 1 à 3 atomes de carbone ou un radical benzyl, $R_1$ et $R_2$ représentent un atome d'hydrogène ou un radical alcoyle linéaire ou ramifié renfermant de 1 à 8 atomes de carbone, éventuellement substitué, ainsi que par leurs sels d'addition avec les acides pharmaceutiquement acceptables.

Parmi ceux-ci, on retient notamment ceux répondant à la formule I, dans laquelle R représente un atome d'hydrogène et $R_1$ et $R_2$ représentent un atome d'hydrogène ou un radical alcoyle linéaire ou ramifié, renfermant de 1 à 8 atomes de carbone, éventuellement substitué par un radical phényl ou phénoxy eux-mêmes, éventuellement substitués par un atome d'halogène, et le pointillé représente une liaison carbone-carbone, ainsi que leurs sels d'addition avec les acides pharmaceutiquement acceptables.

Parmi ces derniers, on retient tout particulièrement, les dérivés dont les noms suivent:

1'α(aminométhyl-1H-indole-4-méthanol,

1'α[[(-méthyléthyl)amino]méthyl-1H-indole-4-méthanol,

1'α(méthylamino)méthyl-1H-indole-4-méthanol,

1'α[[(1-propyl)amino]méthyl]-1H-indole-4-méthanol,

ainsi que leurs sels d'addition avec les acides pharmaceutiquement acceptables.

Les médicaments selon l'invention trouvent leur emploi, par exemple, dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé ou atteint d'artériosclérose, et dans le traitement de l'hypertension d'origine rénale. Ils trouvent également leur emploi dans le traitement des ulcères d'origines diverses.

La dose usuelle, variable selon le produit utilisé, le sujet traité et l'affection en cause, peut être par exemple, de 5 mg à 200 mg par jour par voie orale, chez l'homme, du produit de l'exemple 1.

L'invention a également pour objet les compositions pharmaceutiques qui renferment au moins un dérivé précité, ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médicine humaine, comme, par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits industriels nouveaux utiles notamment pour la préparation des dérivés répondant à la formule I, à savoir les produits de formule III:

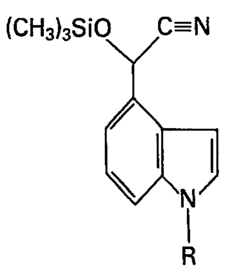

(III)

dans laquelle R a la signification déjà indiquée.

Les produits de formule II peuvent être préparés en faisant réagir le 4-formyl indole avec un halogénure de formule $V_a$:

Hal–R' $(V_a)$

dans laquelle Hal et R' ont la signification déjà indiquée, par exemple, par transfert de phase selon la technique décrite par Bosco et al dans Synthesis 2, 124 (1976), en présence de soude, de benzène et d'un sel d'ammonium quaternaire tel que le chlorure de tétrabutylammonium ou l'hydrogénosulfate de tétrabutylammonium.

Il va être donné maintenant, à titre non limitatif, des exemples de mise en œuvre de l'invention:

Exemple 1

Fumarate de 1α-(aminométhyl)-1H-indole-4-méthanol

On agite pendant 30 mn sous atmosphère inerte 1 ml de cyanure de triméthylsilyle avec 1 g de 4-formyl indole et une quantité catalytique d'iodure de zinc, élimine le cyanure en excès sous pression réduite, ajoute 350 ml d'hydrure d'aluminium lithium en solution dans du tétrahydrofurane, agite pendant 16 heures à 0°C, détruit l'excès d'hydrure par addition de soude aqueuse diluée, filtre, lave au tétrahydrofurane, sèche sur sulfate de sodium, concentre, purifie par chromatographie sur silice (Eluant: acétate d'éthyle-méthanol-ammoniaque 92-5-3) et obtient 0,8 g d'un produit huileux.

Formation du fumarate

On dissout 5,5 g de la base telle qu'obtenue ci-dessus dans 200 $cm^3$ d'isopropanol, ajoute 3,6 g d'acide fumarique chauffe au reflux pendant quinze minutes, concentre à 100 $cm^3$, glace, filtre, sèche à 60°C sous pression réduite, recristallise dans l'isopropanol et obtient 6 g du produit attendu. F = 196°C.

Analyse:

$C_{14}H_{16}N_2O_5$ = 292,294

Calculé: C 57,53% H 5,52% N 9,58%

Trouvé: C 57,8% H 5,4% N 9,4%

Exemple 2

Oxalate neutre de 1'α[[(1-méthyléthyl)amino]méthyl]-1H-indole-4-méthanol

On agite à 50°C, sous hydrogène, 2,8 g d'α-aminométhyl 1H-indole-4-méthanol dans 50 $cm^3$ de méthanol et 20 $cm^3$ d'acétone, en présence de 1 g de palladium à 10% sur charbon, pendant 4 heures. On filtre, chasse les solvants du filtrat sous pression réduite à 50°C, purifie le résidu par chromatographie sur silice (Eluant: chloroforme-acétone'triéthylamine 6-3-1) et obtient 3 g de la base du produit attendu.

Formation de l'oxalate neutre

On dissout le produit ci-dessus dans 200 $cm^3$ d'isopropanol, ajoute 1,73 g d'acide oxalique, l'oxalate formé cristallise. On ajoute 200 $cm^3$ de méthanol, chauffe au reflux jusqu'à dissolution, concentre, glace, filtre, sèche sous pression réduite, recristallise dans l'éthanol et obtient 2,3 g du produit attendu. F = 275°C.

Analyse: $C_{14}H_{19}N_2O_3$ = 263,318

Calculé: C 63,86% H 7,27% N 10,64%

Trouvé: C 63,7% H 7,4% N 10,7%

Exemple 3

α-(méthylamino)méthyl-1H-indole-4-méthanol

Stade A: Formation du carbamate

On ajoute lentement sous atmosphère inerte 4 $cm^3$ de chloroformiate d'éthyle à une solution de 5,40 g d'α-aminométhyl 1H-indole-4-méthanol dans 50 $cm^3$ de méthanol et 5,90 $cm^3$ de triéthylamine, agite pendant 30 mn, concentre, reprend à l'acétate d'éthyle, lave à l'eau, sèche sur sulfate de

sodium, concentre, recristallise dans l'acétate d'éthyle pour obtenir 4,10 g de produit attendu.

Stade B: Réduction du carbamate

On ajoute 2 g d'hydrure d'aluminium lithium à 5,65 g du carbamate ci-dessus, en solution dans 700 $cm^3$ de tétrahydrofurane sous atmosphère inerte, porte pendant 20 heures au reflux, refroidit, détruit l'excès d'hydrure, filtre, lave au tétrahydrofurane, sèche sur sulfate de sodium, concentre, cristallise dans l'acétate d'éthyle, pour obtenir 2,09 g du produit attendu F = 120°C.

Analyse: $C_{11}H_{14}N_2O$ = 190,24
Calculé: C 69,44% H 7,42% N 14,73%
Trouvé: C 69,4% H 7,4% N 14,6%

Exemple 4
α-(aminométhyl)-1-(phénylméthyl)-1H-indole-4-méthanol

a) 1-[1-(phénylméthyl)-1H-indol-4-yl]éthanone

On refroidit à −40°C, 60 $cm^3$ d'anhydride acétique dans 300 $cm^3$ de tétrahydrofuranne anhydre, ajoute sans dépasser −20°C, une solution de magnésien préparée à partir de 150 g de 4-chloro-1-phénylméthyl-1H-indole (décrit dans la demande de brevet français 2458549) et agite pendant 1 heure à −20°C/+10°C. On ajoute une solution saturée de chlorure d'ammonium, dilue à l'eau et extrait à l'acétate d'éthyle. On lave à l'eau salée les phases organiques, sèche, élimine les solvants à 50°C sous pression réduite et purifie le résidu obtenu par chromatographie sur silice (éluant: cyclohexane-acétate d'éthyle (9-1). On recristallise le produit brut dans l'éther isopropylique et obtient 70 g du produit cherché. F = 93°C.

b) α-(aminométhyl)-1-(phénylméthyl)-1H-indole-4-méthanol

On refroidit à 0°C+5°C une solution de 5,5 g du produit ci-dessus dans 60 $cm^3$ de tétrahydrofuranne et 66 $cm^3$ d'une solution N de terbutylate de potassium dans le terbutanol, ajoute 13,1 $cm^3$ de nitrite de terbutyle et maintient sous agitation et atmosphère inerte pendant une heure. On dilue à l'eau, ajoute de l'acide acétique concentré jusqu'à pH: 4,5, agite pendant 15 minutes puis neutralise à l'aide d'une solution aqueuse saturée de bicarbonate de sodium. On extrait à l'acétate d'éthyle et obtient 6,6 g d'oxime.

On refroidit à 0/5°C une suspension de 9 g d'hydrure d'aluminium-lithium dans 120 $cm^3$ de tétrahydrofuranne, ajoute le produit ci-dessus en solution dans 120 $cm^3$ de tétrahydrofuranne et laisse sous agitation pendant 20 heures à 20/25°C. On refroidit à 0/5°C, détruit l'excès d'hydrure, reprend à l'eau et à l'acétate d'éthyle, filtre, extrait à l'acétate d'éthyle, sèche, concentre à sec. On purifie le résidu obtenu par chromatographie sur silice (éluant: chloroforme-méthanol 1-1), recristallise dans le benzène au reflux, et obtient 1,8 g du produit attendu. F = 92°C.

Exemple 5
α-[[(1-méthyléthyl)amino]méthyl]2,3-dihydro-1H-indole-4-méthanol

On agite pendant 20 heures à 20°C, un mélange de 4 g de produit obtenu à l'exemple 2 (base) 60 $cm^3$ de dioxanne, 6,6 g de complexe triméthylamine-borane et 9,2 $cm^3$ d'acide chlorhydrique 12N. On ajoute 600 $cm^3$ d'eau, concentre sous pression réduite à 400 $cm^3$ environ et lave à l'éther. On alcalinise par de la soude, extrait à l'acétate d'éthyle, chromatographie le résidu sur silice en éluant au mélange chloroforme-acétone-triéthylamine (6-3-1) et obtient 3 g de produit attendu. F = 88°C après recristallisation dans l'éther isopropylique.

Analyse: $C_{13}H_{20}N_{20}$ = 220,316
Calculé: C 70,87% H 9,15% N 12,71%
Trouvé: C 71,1% H 9,3% N 12,6%

Exemple 6
α-(méthylamino)métyl-2,3-dihydro-1H-indole-4-méthanol

On opère de manière analogue à celle décrite à l'exemple 5, au départ de 4,4 g de produit obtenu à l'exemple 3 et obtient, après chromatographie sur silice en éluant au mélange acétate d'éthyle-méthanol-ammoniaque (92-5-3), 2,4 g de produit attendu. F = 132°C après recristallisation dans le benzène.

Analyse: $C_{11}H_{16}N_2O$ = 192,263
Calculé: C 68,72% H 8,39% N 14,57%
Trouvé: C 68,6% H 8,2% N 14,4%

Exemple 7
oxalate neutre de 1'α-[[(1-éthylpropyl)amino]méthyl]-1H-indole-4-méthanol

On opère de manière analogue à celle décrite à l'exemple 2, au départ de 3,45 g de dérivé indolique et de 10 $cm^3$ de diéthylcétone. On obtient après chromatographie sur silice en éluant au mélange chloroforme-acétone-triéthylamine (8-1-1), 4,3 g de base du produit attendu.

On forme l'oxalate dans l'isopropanol avec 1 g d'acide oxalique et obtient 3,8 g de produit attendu. F = 212°C après recristallisation dans le méthanol.

Analyse: $C_{32}H_{46}N_4O_6$ = 582,747
Calculé: C 65,96% H 7,96% N 9,61%
Trouvé: C 66,2% H 8,0% N 9,5%

Exemple 8
Oxalate acide de 1'α[[(phénylméthyl)amino]méthyl]-1H-indole-4-méthanol

Stade A: a-[[(phénylcarbonyl)amino]méthyl]-1H-indole-4-méthanol

On mélange 3,6 g de produit obtenu à l'exemple 1 (base), 60 $cm^3$ de tétrahydrofuranne et 5 $cm^3$ de triéthylamine. On refroidit à 0–5°C, ajoute une solution de 3,1 $cm^3$ de chlorure de benzoyle dans 25 $cm^3$ de tétrahydrofuranne et agite pendant 30 minutes à 0°C. On dilue à l'eau, extrait à l'éther et concentre la solution à 40°C à environ 50 $cm^3$.

Stade B: Oxalate acide de 1'α-[[(phénylméthyl) amino]méthyl]-1H-indole-4-méthanol

On dilue la solution obtenue au stade A par du tétrahydrofuranne puis introduit à 0/+5°C, 5 g d'hydrure d'aluminiumlithium et porte au reflux pendant une heure. On refroidit, ajoute du tétrahydrofuranne à 20% d'eau puis de l'eau, filtre et extrait à l'acétate d'éthyle. On évapore le solvant, chromatographie le résidu sur silice en éluant à l'acétate d'éthyle et obtient 4 g de produit attendu.

On dissout le produit dans l'isopropanol au reflux et ajoute 2 g d'acide oxalique. On concentre, refroidit, essore et sèche les cristaux formés. On obtient 4,9 g de produit attendu.

F = 250°C (Décomposition).

Analyse: $C_{19}H_{20}N_2O_5$ = 356,381

Calculé: C 64,04% H 6,66% N 7,86%

Trouvé: C 63,9% H 6,7% N 7,8%

Exemple 9

Oxalate acide de 1'α-[[(2-phényléthyl)amino]méthyl]-1H-indole-4-méthanol

Stade A: α-[[(2-phényl-2-oxo-éthyl)amino]méthyl]-1H-indole-4-méthanol

On opère de manière analogue à celle décrite au stade A de l'exemple 8, en utilisant 4 g de dérivé indolique et 4,5 $cm^3$ de clorure de phénylacétyle. On obtient le produit attendu.

Stade B: Oxalate acide de 1'α[[(2-phényléthyl) amino]méthyl]-1H-indole-4-méthanol

On opère de manière analogue à celle décrite au stade B de l'exemple 8 et obtient 1,9 g de produit attendu (base) puis, après recristallisation dans l'isopropanol, 2 g de sel. F = 200°C.

Analyse: $C_{20}H_{22}N_2O_5$ = 370,408

Calculé: C 64,85% H 5,99% N 7,56%

Trouvé: C 64,8% H 6,0% N 7,6%

Exemple 10

α-[[(2-hydroxy-3-phénoxy-propyl)amino]méthyl]-1H-indole-4-méthanol

Stade A: α-[[(2-hydroxy-3-phénoxypropyl)-(phénylméthyl)amino]méthl]-1H-indole-4-méthanol

On agite pendant 65 heures à 20°C un mélange de 3,7 g de produit obtenu à l'exemple 8 (base), 50 $cm^3$ de méthanol et 3,8 $cm^3$ de 1,2-époxy-3-phénoxypropane. On dilue à l'eau sature au carbonate de potassium et extrait à l'acétate d'éthyle. On sèche et évapore le solvant. On chromatographie le résidu sur silice en éluant au mélange benzène-acétate d'éthyle (7-3) et obtient 5,25 g de produit attendu.

Stade B: α-[[(2-hydroxy-3-phénoxypropyl)amino]-méthyl]-1H-indole-4-méthanol

On mélange le produit obtenu au stade A avec 100 $cm^3$ de méthanol et 2,5 g de palladium à 10% sur charbon et maintient sous agitation sous hydrogène à 40°C jusqu'à fin d'absorption. On filtre, chasse le solvant sous pression réduite et chromatographie le résidu sur silic en éluant au mélange chloroforme-méthanol-triéthylamin (9-0,5-0,5). On obtient 3,1 g de produit attendu.

Analyse: $C_{19}H_{22}N_2O_3$ = 326,398

Calculé: C 69,92% H 6,79% N 8,58%

Trouvé: C 69,7% H 6,9% N 8,5%

Exemple 11

Oxalate de 1'α[[(1-propyl)amino]méthyl]-1H-indole-4-méthanol

Stade A: α-[[(1-oxo-1-propyl)amino]méthyl-1H-indole-4-méthanol

On mélange 15 g de produit obtenu à l'exemple 1, 250 $cm^3$ de tétrahydrofuranne et 3 équivalents de triéthylamine. On ajoute à 0,5°C, 4,9 $cm^3$ de chlorure de propionyle dans 100 $cm^3$ de tétrahydrofuranne et maintient à 0/+5°C pendant une heure. On ajoute de l'eau, extrait à l'éther et chromatographie sur silice en éluant au mélange chloroforme-acétone-triéthylamine (6-3-1). On obtient 8 g de produit attendu.

Stade B: Oxalate de 1'α-[[(1-propyl)amino]méthyl]-1H-indole-4-méthanol

On dissout le produit obtenu au stade A dans 300 $cm^3$ de tétrahydrofuranne et ajoute 1 équivalent d'hydrure d'aluminium-lithium. On porte au reflux pendant une heure trente, refroidit, ajoute du tétrahydrofuranne à 20% d'eau puis de l'eau, filtre, extrait à l'acétate d'éthyle. On évapore le solvant et chromatographie le produit sur silice en éluant au mélange chloroforme-méthanol (7-3).

On dissout le produit dans l'isopropanol au reflux, ajoute un équivalent d'acide oxalique, concentre, refroidit, essore les cristaux formés et les recristallise dans le mélange éthanol/méthanol (1-1). On obtient 6 g de produit attendu. F = 202°C.

Exemple 12

Fumarate neutre de 1'α-[[di-(1-propyl)amino]méthyl]-1H-indole-4-méthanol

On mélange 10,6 g de produit obtenu à l'exemple 1 (base), 200 $cm^3$ de méthanol, 8,8 $cm^3$ de propionaldéhyde et 3 g de palladium à 10% sur charbon et maintient sous agitation et sous hydrogène à 40°C jusqu'à fin d'absorption. On filtre, chasse le solvant sous pression réduite et chromatographie le résidu sur silice en éluant au mélange cyclohexane-chloroforme-triéthylamine (6-3-1). On obtient après recristallisation dans l'éther isopropylique, 9,1 g de produit attendu (base). F = 78°C.

On dissout 4,3 g du produit ci-dessus dans 100 $cm^3$ d'isopropanol et ajoute, au reflux, 1,9 g d'acide fumarique.

On refroidit, essore le sel, le sèche et le recristallise dans le mélange isopropanol-méthanol (2-1). On obtient 3,8 g de sel attendu. F = 174°C.

Analyse: $C_{18}H_{26}N_2O_3$ = 318,419

Calculé: C 67,90% H 8,23% N 8,80%

Trouvé: C 67,9% H 8,3% N 8,6%

Exemple 13

α-[[(2-hydroxy-3-(4-acétylaminophénoxy)-propyl]amino]méthyl]-1H-indole-4-méthanol

Stade A: α-[[(2-hydroxy-3-(4-acétylaminophénoxy)-propyl]]phénylméthyl]amino]méthyl]-1H-indole-4-méthanol

On opère de manière analogue à celle décrite à l'exemple 10, en utilisant 5,6 g de dérivé indolique et 8,7 g de 1,2-époxypropoxy acétanilide (décrit dans J. Pharm. et Pharmacol. 5 359, 1953). On obtient après purification par chromatographie sur silice, en éluant au mélange chloroforme-méthanol (9-1), 7,6 g de produit attendu.

Stade B: α-[[(2-hydroxy-3-(4-acétylaminophénoxy)-propyl]amino]méthyl]-1H-indole-4-méthanol

On opère de manière analogue à celle décrite à l'exemple 10, et obtient au départ de 7,6 g de produit du stade A, et après chromatographie sur silice en éluant au mélange chloroforme-méthanol-triéthylamine (8-1-1), 3,9 g de produit attendu.

Analyse: $C_{21}H_{25}N_3O_4$ = 383,451

Calculé: C 65,78% H 6,57% N 10,96%

Trouvé: C 65,3% H 6,8% N 10,8%

Exemple 14

On a préparé des comprimés répondant à la formule:

Oxalate neutre de 1'α[[(1-méthyléthyl)amino] méthyl]-1H-indole-4-méthanol 10 mg

Excipient q.s. pour un comprimé terminé à 100 mg

(Détail de l'excipient: lactose, amidon, talc, stéarate de magnésium).

Exemple 15

On a préparé des comprimés répondant à la formule:

α-(méthylamino)méthyl-1H-indole-4-méthanol 20 mg

exipient q.s. pour un comprimé terminé à 100 mg

(Détail de l'excipient: lactose, amidon, talc, stéarate de magnésium).

Etude pharmacologique:

A) Détermination de l'activité hypotensive

L'activité hypotensive a été étudiée sur des rats mâles de souche WISTAR pensant 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témoin initiale:

| Produit de l'exemple | Dose en mg/kg | Variation % de la pression arterielle | | | |
|---|---|---|---|---|---|
| | | 1 minute après l'administration | 5 minutes après l'administration | 10 minutes après l'administration | 30 minutes aprés l'administration |
| 2 | 1 | −41 | −38 | −29 | −20 |
| | 0,1 | −36 | −28 | −30 | −27 |
| 3 | −0,1 | +19 | − 4 | − 5 | − 9 |
| 5 | 1 | −20 | −19 | −17 | −14 |
| | 0,1 | −24 | −28 | −26 | −22 |
| 7 | 10 | −59 | −56 | −50 | −35 |
| 8 | 10 | −38 | −21 | −21 | −15 |
| | 1 | − 9 | − 8 | − 6 | −10 |
| 9 | 10 | −48 | −44 | −37 | −33 |
| | 1 | −13 | −11 | − 4 | − 3 |
| 10 | 1 | −32 | −14 | −15 | −13 |
| 11 | 1 | −22 | −33 | −33 | −30 |
| | 0,1 | −11 | −12 | −12 | −13 |

B) Détermination de l'activité antihypertensive

L'action antihypertensive a été étudiée sur des rats mâles spontanément hypertendus (souche Okamoto) âgés de 20 semaines pesant 300 à 320 g.

Le produit a été administré par voie orale 48 heures après la pose d'un cathéter intracarotidien.

La pression artérielle a été mesurée à la queue du rat au moyen d'un manchon pneumatique et à l'aide d'un transducteur piezo-électrique de pression. La pression a été mesurée avant et une heure, quatre heures et vingt-quatre heures après l'administration du produit.

Le tableau ci-après indique les variations exprimées en pourcentages de la pression artérielle, après administration du produit, par rapport à la pression témoin initiale.

| Produit de l'exemple | Dose en mg/kg | Variation % de la pression arterielle | | |
|---|---|---|---|---|
| | | 1 heure après l'adminis-tration | 4 heures après l'adminis-tration | 24 heures après l'adminis-tration |
| 2 | 1 | −22 | −21 | − 5 |
| 5 | 1 | −27 | −24 | −17 |

C) Détermination de l'activité antiulcéreuse

La technique utilisée est décrite par SHAY et al dans Gastroenterology, 5, 43 (1945).

La technique de SHAY consiste à induire chez ;z des rats des ulcéres au niveau de l'estomac, par ligature du pylore.

Les animaux sont anesthésiés à l'éther. Une incision longitudinale est faite 1 cm environ au-dessous du sternum, la partie glandulaire de l'estomac et le duodénum sont mis à jour et une ligature est posée quelques mm au-dessous du pylore. Le plan musculaire est laissé tel quel et la peau est suturée par deux agrafes.

Les animaux reçoivent aussitôt après le dispersif ou la substance à étudier, par voie buccale, sous un volume de 0,5 ml/100 g, et sont maintenus sans nourriture ni boisson, jusqu'au sacrifice par saignée carotidienne qui a lieu environ 16 heures après le traitement.

Avant de prélèver l'estomac, une ligature est possé au-dessus du cardia.

Le liquide gastrique est recueilli afin d'en mesure le pH.

L'estomac est ensuite ouvert selon la grande courbure, rincé dans le sérum physiologique et étalé sur du papier millimétrique pour être examiné sous la loupe binoculaire.

On évalue macroscopiquement la gravité des lésions qui est côtée de 0 à 4 pour chaque estomac.

On détermine pour chaque lot de rat, l'intensité moyenne des ulcérations et on calcule la protection en rapportant l'index moyen du groupe à l'index moyen du groupe témoin.

On détermine également les valeurs de pH du liquide gastrique pour les animaux traités et les animaux témoins.

On a obtenu les résultats suivants:

| Produit de l'exmple | Dose (mg/kg) | pH du liquide gastrique animaux traités | animaux témoins | Ulcération % de protection par rapport aux témoins |
|---|---|---|---|---|
| 5 | 2 | 2,6 | 2 | 90 % |
| | 0,4 | 2,5 | 2,2 | 60 % |
| | 0,08 | 2,6 | 2,2 | 66 % |
| 11 | 5 | 2,9 | 3,0 | 63 % |
| | 1 | 3,2 | 3,3 | 48 % |

D) Etude de la toxicité aigue

On a évalué les doses létales $DL_0$ des différents composés testés après administration par voie orale chez la souris.

On apelle $DL_0$ la dose maximale ne provoquant aucune mortalité en 8 jours.

Les résultats obtenus obtenus sont les suivants:

| Produit de l'exemple | $DL_o$ en mg/kg |
|---|---|
| 1 | >1000 |
| 2 | 200 |
| 3 | 600 |
| 5 | 200 |
| 6 | > 400 |
| 7 | 80 |
| 8 | 100 |
| 9 | 80 |
| 10 | > 400 |
| 11 | 100 |

**Revendications pour les Etats Contractants: BE, CH, DE, FR, GB, IT, LI, LU, NL, SE**

1. Les dérivés de l'aminométhyl 1H-indol-4-méthanol ainsi que leurs sels d'addition avec les acides minéraux ou organiques, caractérisés en ce qu'ils répondent à la formule générale I:

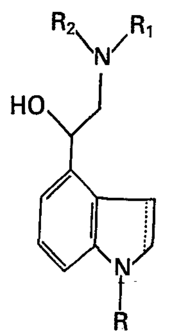

(I)

dans laquelle R représente un atome d'hydrogène, ou un radical alcoyle renfermant de 1 à 6 atomes de carbone ou phényl alcoyle renfermant de 7 à 10 atomes de carbone et $R_1$ et $R_2$, identiques

ou différents, représentent un atome d'hydrogène, un radical alcoyle linéaire ou ramifié renfermant de 1 à 8 atomes de carbone, éventuellement substitué, sauf dans le cas du radical méthyle par un ou plusieurs atomes d'halogène ou par un ou plusieurs radicaux hydroxy, phényle ou phénoxy eux-mêmes éventuellement substitués par un ou plusieurs atomes d'halogène ou par un ou plusieurs radicaux hydroxy, méthyle, méthoxy ou acetamido, $R_1$ et $R_2$ peuvent également représenter un radical cycloalcoyle renfermant de 3 à 7 atomes de carbone, un radical cycloalcoylalcoyle renfermant de 4 à 7 atomes de carbone ou un radical allyle ou propargyle et le pointillé représente la présence éventuelle d'une liaison carbone-carbone.

2. Les dérivés de l'aminométhyl 1H-indol-4-méthanol, tels que définis par la formule I de la revendication 1, ainsi que leurs sels d'addition avec les acides minéraux ou organiques, caractérisés en ce que R représente un atome d'hydrogène, un radical alcoyle renfermant de 1 à 3 atomes de carbone ou un radical benzyle, $R_1$ et $R_2$ représentent un atome d'hydrogène ou un radical alcoyle, linéaire ou ramifié, renfermant de 1 à 8 atomes de carbone, éventuellement substitué.

3. Les dérivés de l'aminométhyl 1H-indol-4-méthanol selon la revendication 2, ainsi que leurs sels d'addition avec les acides minéraux ou organiques, caractérisés en ce que R représente un atome d'hydrogène, $R_1$ et $R_2$ représentent un atome d'hydrogène ou un radical alcoyle linéaire ou ramifié renfermant de 1 à 8 atomes de carbone, éventuellement substitué par un radical phényle ou phénoxy, eux-mêmes éventuellement substitués par un atome d'halogène et le pointillé représente une liaison carbone-carbone.

4. L'un quelconque des dérivés selon la revendication 1 dont les noms suivent ainsi que leurs sels d'addition avec les acides minéraux ou organiques:

1'α-(aminométhyl)-1H-indol-4-méthanol,

1'α-[[(méthyléthyl)aminoy]méthyl]-1H-indol-4-méthanol,

1'α-(méthylamino)méthyl-1H-indol-4-méthanol,

1'α-[[(1-propyl)amino]méthyl]-1H-indol-4-méthanol.

5. Procédé de préparation des dérivés répondant à la formule I de la revendication 1 ainsi que de leurs sels d'addition avec les acides minéraux ou organiques, caractérisé en ce que l'on fait réagir un produit de formule II:

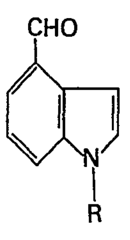

(II)

dans laquelle R a la signification déjà indiquée, avec le cyanure de triméthyl silyle, pour obtenir un produit de formule III:

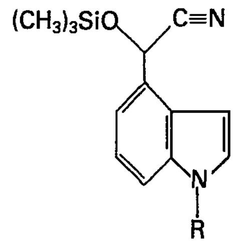

(III)

dans laquelle R a la signification déjà indiquée, que l'on réduit, pour obtenir un produit de formule $I_A$:

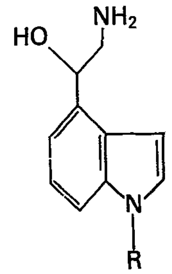

($I_A$)

dans laquelle R a la signification déjà indiquée que:

soit l'on isole et, si désiré, salifie,

soit l'on fait réagir ledit produit de formule $I_A$ avec un dérivé carbonylé de formule IV:

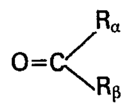

(IV)

dans laquelle Rα et Rβ sont tels que: 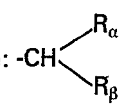

a la signification de $R_1$ déjà indiquée à l'exception d'un atome d'hydrogène et du radical méthyle, en présence d'un agent de réduction, pour obtenir un produit de formule $I_B$:

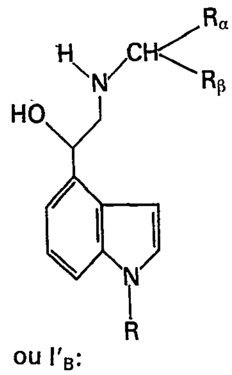

($I_B$)

ou $I'_B$:

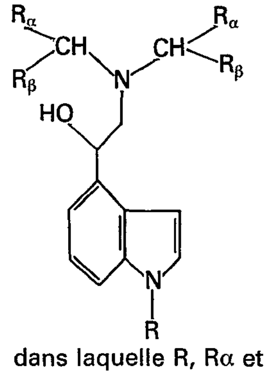

($I'_B$)

dans laquelle R, Rα et Rβ ont la signification déjà indiquée, produit de formule $I_B$ ou $I'_B$ que l'on isole et, si désiré, salifie ou produit de formule $I_B$ que

l'on soumet à l'action d'un halogénure de formule V:

$X–R'_2$ (V)

dans laquelle X représente un atome de chlore, de brome ou d'iode, et $R'_2$ a la signification de $R_2$ déjà indiquée à l'exception de l'hydrogène, pour obtenir un produit de formule $I_C$:

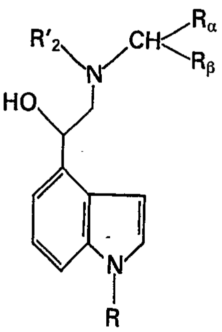

($I_C$)

dans laquelle R, Rα, Rβ et $R'_2$ ont la signification déjà indiquée, que l'on isole et, si désiré, salifie, soit l'on fait réagir ledit produit de formule $I_A$ avec un dérivé carbonylé de formule $IV_a$:

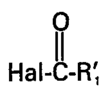

($IV_a$)

dans lequel Hal représente un atome de chlore, de brome ou d'iode, et $-\underset{\underset{O}{\|}}{C}-R'_1$ est tel que: $-CH_2-R'_1$ a la signification de $R_1$ déjà indiquée, à l'exception d'un atome d'hydrogène et du radical méthyle, puis soumet le dérivé obtenu à l'action d'un agent de réduction, pour obtenir un produit de formule $I_D$:

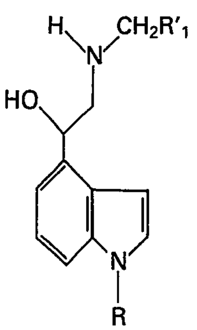

($I_D$)

dans laquelle R et $R'_1$ ont la signification déjà indiquée, produit de formule $I_D$ que ou bien l'on isole et, si désiré, salifie, ou bien l'on soumet à l'action d'un halogénure de formule V:

$X–R'_2$ (V)

dans laquelle X et $R'_2$ ont la signification déjà indiquée, pour obtenir un produit de formule $I_E$:

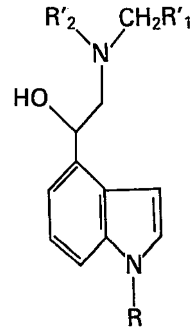

($I_E$)

dans laquelle R, $R'_1$ et $R'_2$ ont la signification déjà indiquée, que l'on isole et, si désiré, salifie, soit l'on soumet ledit produit de formule $I_A$ à une méthylation, pour obtenir un produit de formule $I_F$:

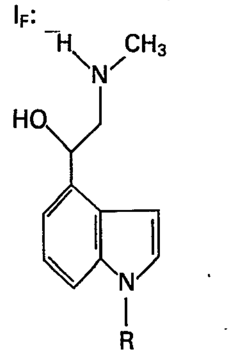

($I_F$)

dans laquelle R a la signification déjà indiquée, que ou bien l'on isole et, si désiré, salifie, ou bien l'on fait réagir avec un halogénure de formule V défini ci-dessus, pour obtenir produit de formule $I_G$:

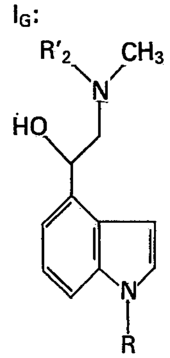

($I_G$)

dans laquelle R et $R'_2$ ont la signification déjà indiquée, que l'on isole et, si désiré, salifie, et si désiré, soumet lesdits produits de formules $I_A$, $I_B$, $I'_B$, $I_C$, $I_D$, $I_E$, $I_F$ et $I_G$, l'action d'un agent de réduction du noyau indole, pour obtenir un produit de formule I':

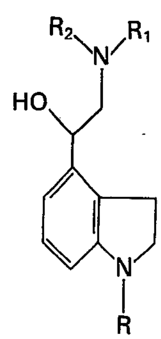

(I')

dans laquelle R, $R_1$ et $R_2$ ont la signification déjà indiquée, que l'on isole et, si désiré, salifie.

6. Variante du procédé de préparation selon la revendication 5, caractérisée en ce que l'on fait réagir le 4-chloro-indole avec un halogénure de formule $V_a$:

Hal–R' $(V_a)$

dans laquelle Hal représente un atome de chlore, de brome ou d'iode et R' a la signification de R déjà indiquée, à l'exception de l'hydrogène, pour obtenir un produit de formule VI:

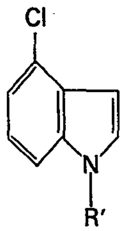
(VI)

dans laquelle R' a la signification déjà indiquée, que l'on transforme en magnésien correspondant, sur lequel on fait réagir un agent d'acétylation, pour obtenir un produit de formule VII:

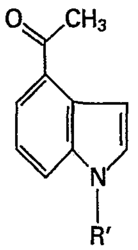
(VII)

dans laquelle R' a la signification déjà indiquée, dont on prépare l'oxime en α de la cétone, puis réduit le produit obtenu, pour obtenir un produit de formule $I'_A$:

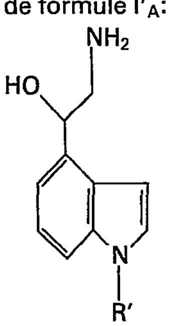
$(I'_A)$

dans laquelle R' a la signification déjà indiquée, que soit l'on isole et, si désiré, salifie, soit poursuit le procédé comme indiqué à la revendication 5 pour le produit de formule $I_A$.

7. Variante du procédé selon la revendication 5, pour la préparation des produits de formule I, dans laquelle $R_1$ représente un radical alcoyle, linéaire ou ramifié renfermant de 2 à 8 atomes de carbone, substitué par un ou plusieurs radicaux hydroxy dont au moins un hydroxy sur le carbone en position 2 et, en outre, éventuellement substitué par un ou plusieurs atomes d'halogène ou par un ou plusieurs radicaux aryle ou aryloxy éventuellement substitués sur le noyau aryle et $R_2$ représente un atome d'hydrogène ou un radical alcoyle renfermant de 1 à 8 atomes de carbone, éventuellement substitué par un radical aryle, variante caractérisée en ce que l'on traite un produit de formule $I_H$:

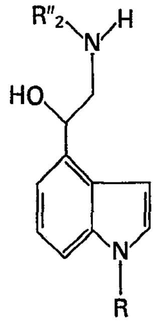
$(I_H)$

dans laquelle R est défini comme ci-dessus et $R''_2$ représente un radical alcoyle renfermant de 1 à 8 atomes de carbone, éventuellement substitué par un radical aryle correspondant à un produit de formule $I_D$ telle que définie précédemment, dans laquelle $CH_2$-$R'_1$ a la signification de $R''_2$ indiquée ci-dessus, par un réactif de formule VIII:

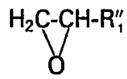
(VIII)

dans laquelle $R''_1$ est tel que $-CH_2-\underset{OH}{CH}-R''_1$ a la valeur de $R_1$ indiquée ci-dessus, pour obtenir un produit de formule $I_I$:

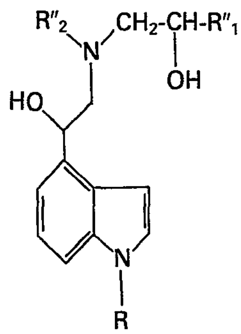
$(I_I)$

que, le cas échéant, l'on soumet à l'action d'un agent de réduction, pour obtenir un composé de formule $I_J$:

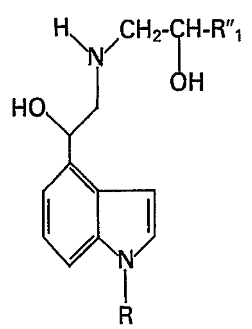
$(I_J)$

que soit l'on isole et, si désiré, salifie,
soit l'on soumet, comme les produits de formule $I_B$, $I_D$ ou $I_F$, à l'action d'un halogénure de formule V telle que définie précédemment, pour obtenir un produit de formule $I_K$:

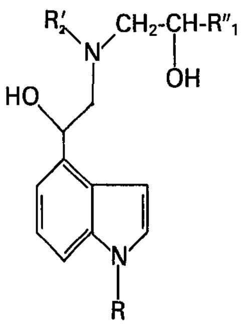

($I_K$)

que l'on isole et, si désiré, salifie et, si désiré, soumet les produits de formule $I_I$, $I_J$ ou $J_K$ à l'action d'un agent de réduction du noyau indole, pour obtenir le produit réduit correspondant que l'on isole et, si désiré, salifie.

8. Médicaments, caractérisés en ce qu'ils sont constitués par les nouveaux dérivés de l'aminométhyl 1H-indol-4-méthanol, tels que définis par la formule I de la revendication 1, ainsi que par leurs sels d'addition avec les acides pharmaceutiquement acceptables.

9. Médicaments, caractérisés en ce qu'ils sont constitués par les nouveaux dérivés de l'aminométhyl 1H-indol-4-méthanol, tels que définis dans l'une des revendications 2 et 3 ainsi que par leurs sels d'addition avec les acides pharmaceutiquement acceptables.

10. Médicaments, caractérisés en ce qu'ils sont constitués par les dérivés de l'aminométhyl 1H-indol-4-méthanol tels que définis à la revendication 4 ainsi que par leurs sels d'addition avec les acides pharmaceutiquement acceptables.

11. Compositions pharmaceutiques, caractérisées en ce qu'elles renferment, à titre de principe actif, l'un au moins des médicaments, tels que définis à l'une des revendications 8, 9 ou 10.

12. A titre de produits industriels nouveaux, les produits de formule III:

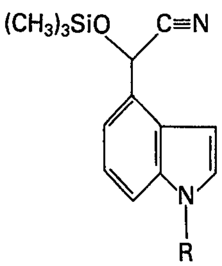

(III)

dans laquelle R a la signification déjà indiquée.

**Revendications pour L'Etat contractant: AT**

1. Procédé pour préparer les dérivés de l'aminométhyl 1H-indol-4-méthanol, ainsi que leurs sels d'addition avec les acides minéraux ou organiques, répondant à la formule générale I:

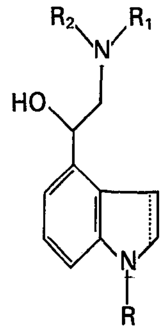

(I)

dans laquelle R représente un atome d'hydrogène, ou un radical alcoyle renfermant de 1 à 6 atomes de carbone ou phényl alcoyle renfermant de 7 à 10 atomes de carbone et $R_1$ et $R_2$, identiques ou différents, représentent un atome d'hydrogène, un radical alcoyle linéaire ou ramifié renfermant de 1 à 8 atomes de carbone, éventuellement substitué, sauf dans le cas du radical méthyle par un ou plusieurs atomes d'halogène ou par un ou plusieurs radicaux hydroxy, phényle ou phénoxy eux-mêmes éventuellement substitués par un ou plusieurs atomes d'halogène ou par un ou plusieurs radicaux hydroxy, méthyle, méthoxy ou acétamido, $R_1$ et $R_2$ peuvent également représenter un radical cycloalcoyle renfermant de 3 à 7 atomes de carbone, un radical cycloalcoylalcoyle renfermant de 4 à 7 atomes de carbone, ou un radical allyle ou propargyle et le pointillé représente la présence éventuelle d'une liaison carbone-carbone, caractérisé en ce que l'on fait réagir un produit de formule II:

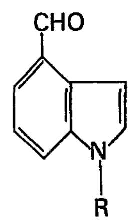

(II)

dans laquelle R a la signification déjà indiquée, avec le cyanure de triméthyl silyle, pour obtenir un produit de formule III:

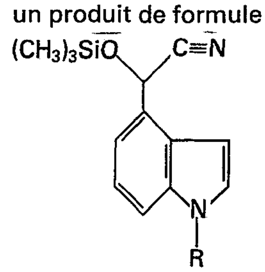

(III)

dans laquelle R a la signification déjà indiqué, que l'on réduit, pour obtenir un produit de formule $I_A$:

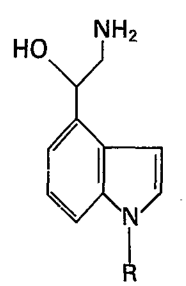

($I_A$)

dans laquelle R a la signification déjà indiquée que:

soit l'on isole et, si désiré, salifie,

soit l'on fait réagir ledit produit de formule $I_A$ avec un dérivé carbonylé de formule IV:

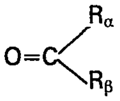

(IV)

dans laquelle Rα et Rβ sont tels que: 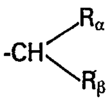 a la signification de $R_1$ déjà indiquée à l'exception d'un atome d'hydrogène et du radical méthyle, en présence d'un agent de réduction, pour obtenir un produit de formule $I_B$:

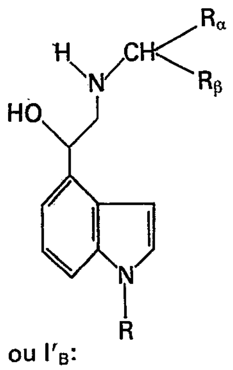

($I_B$)

ou $I'_B$:

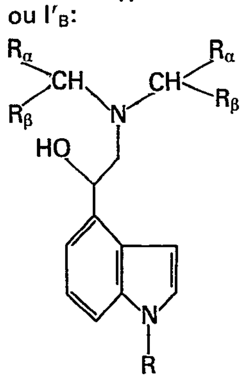

($I'_B$)

dans laquelle R, Rα et Rβ ont la signification déjà indiquée, produit de formule $I_B$ ou $I'_B$ que l'on isole et, si désiré, salifie ou produit de formule $I_B$ que l'on soumet à l'action d'un halogénure de formule V:

$X{-}R'_2$ (V)

dans laquelle X représente un atome de chlore, de brome ou d'iode, et $R'_2$ a la signification de $R_2$ déjà indiquée à l'exception de l'hydrogène, pour obtenir un produit de formule $I_C$:

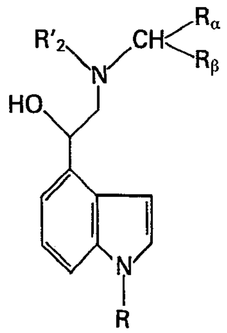

($I_C$)

dans laquelle R, Rα, Rβ et $R'_2$ ont la signification déjà indiquée, que l'on isole et, si désiré, salifie.

soit l'on fait réagir ledit produit de formule $I_A$ avec un dérivé carbonylé de formule $IV_a$:

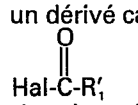

($IV_a$)

dans lequel Hal représente un atome de chlore, de brome ou d'iode, et 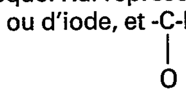 est tel que: $-CH_2-R'_1$ a la signification de $R_1$ déjà indiquée, à l'exception d'un atome d'hydrogène et du radical méthyle, puis soumet le dérivé obtenu à l'action d'un agent de réduction, pour obtenir un produit de formule $I_D$:

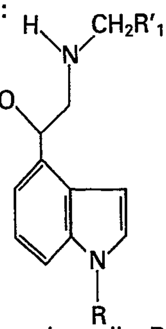

($I_D$)

dans laquelle R et $R'_1$ ont la signification déjà indiquée, produit de formule $I_D$ que ou bien l'on isole et, si désiré, salifie, ou bien l'on soumet à l'action d'un halogénure de formule V:

$X{-}R'_2$ (V)

dans laquelle X et $R'_2$ ont la signification déjà indiquée, pour obtenir un produit de formule $I_E$:

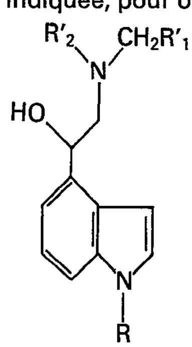

($I_E$)

dans laquelle R, $R'_1$ et $R'_2$ ont la signification déjà indiquée, que l'on isole et, si désiré, salifie, soit l'on soumet ledit produit de formule $I_A$ à une méthylation, pour obtenir un produit de formule $I_F$:

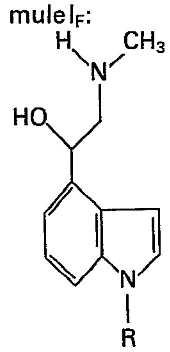

($I_F$)

dans laquelle R a la signification déjà indiquée, que ou bien l'on isole et, si désiré, salifie, ou bien l'on fait réagir avec un halogénure de formule V défini ci-dessus, pour obtenir un produit de formule $I_G$:

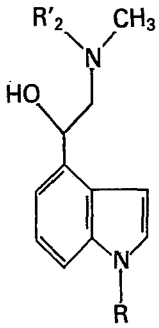

($I_G$)

dans laquelle R et $R'_2$ ont la signification déjà indiquée, que l'on isole et, si désiré, salifie, et si désiré, soumet lesdits produits de formules $I_A$, $I_B$, $I'_B$, $I_C$, $I_D$, $I_E$, $I_F$ et $I_G$, l'action d'un agent de réduction du noyau indole, pour obtenir un produit de formule I':

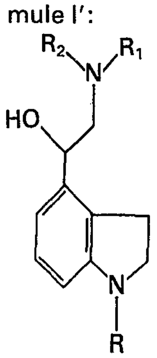

(I')

dans laquelle R, $R_1$ et $R_2$ ont la signification déjà indiquée, que l'on isole et, si désiré, salifie.

2. Procédé selon la revendication 1, caractérisé en ce que l'on fait réagir un produit de formule II:

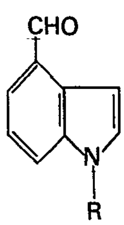

(II)

dans laquelle R a la signification déjà indiquée, avec le cyanure de triméthyl silyle, pour obtenir un produit de formule III:

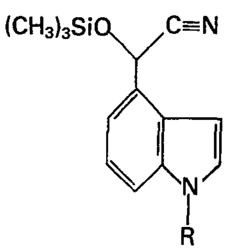

(III)

dans laquelle R a la signification déjà indiquée, que l'on réduit, pour obtenir un produit de formule $I_A$:

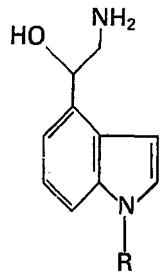

($I_A$)

dans laquelle R a la signification déjà indiquée, que:

soit l'on isole et, si désiré, salifie;

soit l'on fait réagir ledit produit de formule $I_A$ avec un dérivé carbonyle de formule IV:

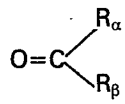

(IV)

dans laquelle Rα et Rβ sont tels que: 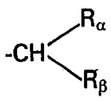

a la signification de $R_1$ déjà indiquée, à l'exception d'un atome d'hydrogène et du radical méthyle, en présence d'un agent de réduction, pour obtenir un produit de formule $I_B$:

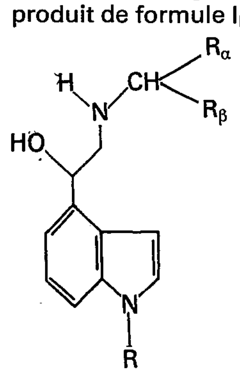

($I_B$)

dans laquelle R, Rα et Rβ ont la signification déjà indiquée, produit de formule $I_B$ que ou bien l'on isole et, si désiré, salifie, ou bien l'on soumet à l'action d'un halogénure de formule V:

$X–R'_2$ (V)

dans laquelle X représente un atome de chlore, de brome ou d'iode, et $R'_2$ a la signification de $R_2$ déjà indiquée, à l'exception de l'hydrogène, pour obtenir un produit de formule $I_C$:

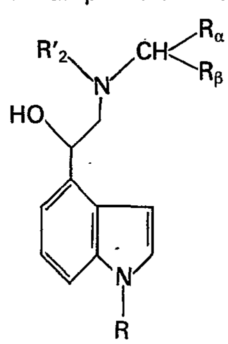

($I_C$)

dans laquelle R, Rα, Rβ et $R'_2$ ont la signification déjà indiquée, que l'on isole, et, si désiré, salifie, soit l'on soumet ledit produit de formule $I_A$ à une méthylation, pour obtenir un produit de formule $I_F$:

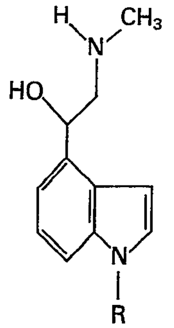

($I_F$)

dans laquelle R a la signification déjà indiquée, que ou bien l'on isole et, si désiré, salifie, ou bien l'on fait réagir avec un halogénure de formule V défini ci-dessus, pour obtenir un produit de formule $I_G$:

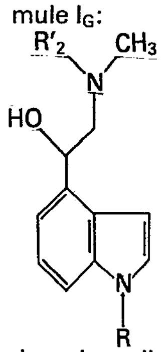

($I_G$)

dans laquelle R et $R'_2$ ont la signification déjà indiquée, que l'on isole et, si désiré, salifie, et, si désiré, soumet lesdits produits de formules $I_A$, $I_B$, $I_C$, $I_F$ et $I_G$ à l'action d'un agent de réduction du noyau indole, pour obtenir un produit de formule I':

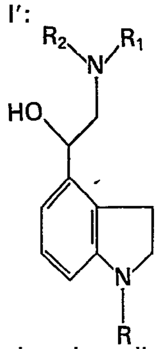

(I')

dans laquelle R, $R_1$ et $R_2$ ont la signification déjà indiquée que l'on isole, et, si désiré, salifie.

3. Variante du procédé de préparation selon la revendication 1, caractérisée en ce que l'on fait réagir le 4-chloro-indole avec un halogénure de formule $V_a$:

Hal–R' ($V_a$)

dans laquelle Hal représente un atome de chlore, de brome ou d'iode et R' a la signification de R déjà indiquée à l'exception de l'hydrogène, pour obtenir un produit de formule VI:

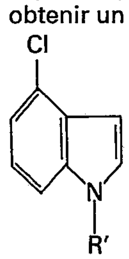

(VI)

dans laquelle R' a la signification déjà indiquée, que l'on transforme en magnésien correspondant, sur lequel on fait réagir un agent d'acétylation, pour obtenir un produit de formule VII:

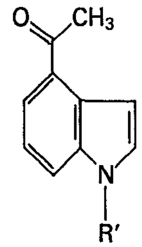

(VII)

dans laquelle R' a la signification déjà indiquée, dont on prépare l'oxime en $\alpha$ de la cétone, puis réduit le produit obtenu, pour obtenir un produit de formule $I'_A$:

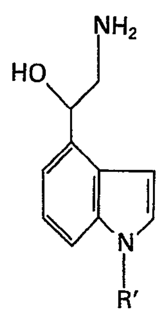

($I'_A$)

dans laquelle R' a la signification déjà indiquée, que soit l'on isole et, si désiré, salifie, soit poursuit le procédé comme indiqué à la revendication 1 pour le produit de formule $I_A$.

4. Variante du procédé selon la revendication 1, pour la préparation des produits de formule I, dans laquelle $R_1$ représente un radical alcoyle, linéaire ou ramifié renfermant de 2 à 8 atomes de carbone, substitué par un ou plusieurs radicaux hydroxy dont au moins un hydroxy sur le carbone en position 2 et, en outre, éventuellement substitué par un ou plusieurs atomes d'halogène ou par un ou plusieurs radicaux aryle ou aryloxy éventuellement substitués sur le noyau aryle et $R_2$ représente un atome d'hydrogène ou un radical alcoyle renfermant de 1 à 8 atomes de carbone, éventuellement substitué par un radical aryle, variante caractérisée en ce que l'on traite un produit de formule $I_H$:

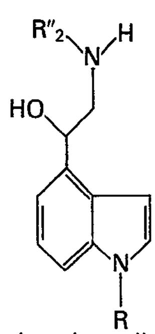

($I_H$)

dans laquelle R est défini comme ci-dessus et $R''_2$ représente un radical alcoyle renfermant de 1 à 8

atomes de carbone, éventuellement substitué par un radical aryle correspondant à un produit de formule $I_D$ telle que définie précédemment, dans laquelle $CH_2-R'_1$ a la signification de $R''_2$ indiquée ci-dessus, par un réactif de formule VIII:

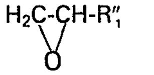
(VIII)

dans laquelle $R''_1$ est tel que $-CH_2-\underset{OH}{CH}-R_1$

a la valeur de $R_1$ indiquée ci-dessus, pour obtenir un produit de formule $I_I$:

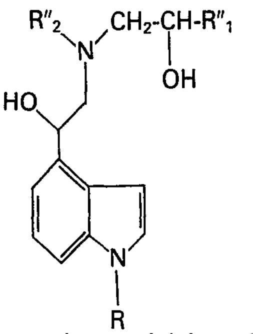
($I_I$)

que, le cas échéant, l'on soumet à l'action d'un agent de réduction, pour obtenir un composé de formule $I_J$:

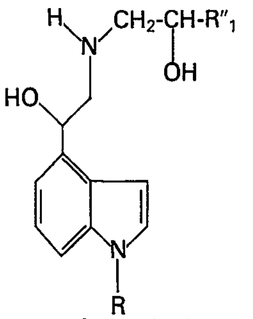
($I_J$)

que soit l'on isole et, si désiré, salifie,
soit l'on soumet, comme les produits de formule $I_B$, $I_D$ ou $I_F$, à l'action d'un halogénure de formule V telle que définie précédemment, pour obtenir un produit de formule $I_K$:

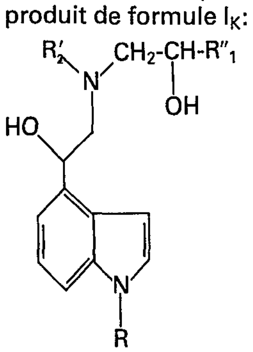
($I_K$)

que l'on isole et, si désiré, salifie et, si désiré, soumet les produits de formule $I_I$, $I_J$ ou $I_K$ à l'action d'un agent de réduction du noyau indole, pour obtenir le produit réduit correspondant que l'on isole et, si désiré, salifie.

5. Procédé selon l'une quelconque des revendications 1 à 4, caractérisé en ce que l'on prépare des produits de formule I, telle que définie à la revendication 1, dans laquelle R représente un atome d'hydrogène, un radical alcoyle renfermant de 1 à 3 atomes de carbone ou un radical benzyle, $R_1$ et $R_2$ représentent un atome d'hydrogène ou un radical alcoyle linéaire ou ramifié, renfermant de 1 à 8 atomes de carbone, éventuellement substitué ainsi que leurs sels d'addition avec les acides minéraux ou organiques.

6. Procédé selon l'une quelconque des revendications 1 à 3, caractérisé en ce que l'on prépare des produits de formule I, telle que définie à la revendication 1, dans laquelle R représente un atome d'hydrogène, $R_1$ et $R_2$ représentent un atome d'hydrogène ou un radical alcoyle, linéaire ou ramifié, renfermant de 1 à 8 atomes de carbone, éventuellement substitué par un radical phényle ou phénoxy, eux-mêmes éventuellement substitués par un atome d'halogène, et le pointillé représente une liaison carbone-carbone ainsi que leurs sels d'addition avec les acides minéraux ou organiques.

7. Procédé selon l'une quelconque des revendications 1 à 3, caractérisé en ce que l'on prépare l'une quelconque des dérivés dont les noms suivent ainsi que leurs sels d'addition avec les acides minéraux ou organiques:

1'α-(aminométhyl)-1H-indol-4-méthanol,

1'α-[[(1-méthyléthyl)amino]méthyl]-1H-indol-4-méthanol,

1'α-(méthylamino)méthyl-1H-indol-4-méthanol,

1'α-[[(1-propyl)amino]méthyl]-1H-indol-4-méthanol.

**Patentansprüche für die Vertragsstaaten: BE, CH, DE, FR, GB, IT, LI, LU, NL, SE**

1. Aminomethyl-1H-indol-4-methanol-Derivate sowie deren Additionssalze mit Mineral- oder organischen Säuren, dadurch gekennzeichnet, dass sie der allgemeinen Formel I

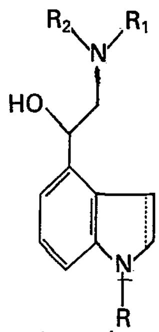
(I)

entsprechen, worin R ein Wasserstoffatom oder einen Alkylrest mit 1 bis 6 Kohlenstoffatomen oder einen Phenylalkylrest mit 7 bis 10 Kohlenstoffatomen bedeutet und $R_1$ und $R_2$ gleich oder verschieden sind und ein Wasserstoffatom, einen linearen oder verzweigten Alkylrest mit 1 bis 8 Kohlenstoffatomen, der gegebenenfalls mit Ausnahme des Falls des Methylrestes durch ein oder mehrere Halogenatome oder durch ein oder mehrere Hydroxy-, Phenyl- oder Phenoxyreste, die ihrerseits gegebenenfalls durch ein oder mehrere Halogenatome oder durch einen oder mehrere Hydroxy-, Methyl-, Methoxy- oder Acetamidoreste substituiert sein können, substituiert ist, bedeuten, $R_1$ und $R_2$ auch einen Cycloalkylrest mit 3 bis 7

Kohlenstoffatomen, einen Cycloalkylalkylrest mit 4 bis 7 Kohlenstoffatomen oder einen Allyl- oder Propargylrest bedeuten können und die gestrichelte Linie die etwaige Anwesenheit einer Kohlenstoff-Kohlenstoff-Bindung anzeigt.

2. Aminomethyl-1H-indol-4-methanol-Derivate der Formel I gemäss Anspruch 1 sowie deren Additionssalze mit Mineral- oder organischen Säuren, dadurch gekennzeichnet, dass R ein Wasserstoffatom, einen Alkylrest mit 1 bis 3 Kohlenstoffatomen oder einen Benzylrest bedeutet, $R_1$ und $R_2$ ein Wasserstoffatom oder einen linearen oder verzweigten Alkylrest mit 1 bis 8 Kohlenstoffatomen, der gegebenenfalls substituiert ist, bedeuten.

3. Amino-methyl-1H-indol-4-methanol-Derivate gemäss Anspruch 2 sowie deren Additionssalze mit Mineral- oder organischen Säuren, dadurch gekennzeichnet, dass R ein Wasserstoffatom bedeutet und $R_1$ und $R_2$ ein Wasserstoffatom oder einen linearen oder verzweigten Alkylrest mit 1 bis 8 Kohlenstoffatomen, der gegebenenfalls durch einen Phenyl- oder Phenoxyrest, der seinerseits gegebenenfalls durch ein Halogenatom substituiert ist, substituiert ist, bedeuten und die gestrichelte Linie eine Kohlenstoff-Kohlenstoff-Bindung anzeigt.

4. Eines der Derivate gemäss Anspruch 1 mit den folgenden Bezeichnungen und deren Additionssalze mit Mineral- oder organischen Säuren
das α-(Aminomethyl)-1H-indol-4-methanol,
das α-[[(1-Methylethyl)-amino]-methyl]-1H-indol-4-methanol,
das α-(Methylamino)-methyl-1H-indol-4-methanol,
das α-[[(1-Propyl)-amino]-methyl]-1H-indol-4-methanol.

5. Verfahren zur Herstellung der Derivate der Formel I gemäss Anspruch 1 sowie von deren Additionssalzen mit Mineral- oder organischen Säuren, dadurch gekennzeichnet, dass man ein Produkt der Formel II

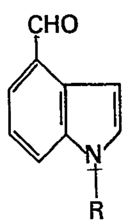

(II)

worin R die angegebene Bedeutung besitzt, mit Trimethylsilylcyanid umsetzt, um ein Produkt der Formel III

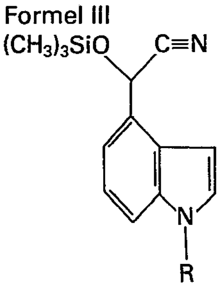

(III)

zu erhalten, worin R die angegebene Bedeutung besitzt, das man reduziert, um ein Produkt der Formel $I_A$

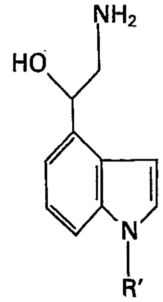

($I'_A$)

zu erhalten, worin R die angegebene Bedeutung besitzt, das
man entweder isoliert und gewünschtenfalls in ein Salz überführt
oder das Produkt der Formel $I_A$ mit einem Carbonylderivat der Formel IV

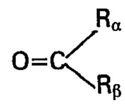

(IV)

worin $R_\alpha$ und $R_\beta$ so beschaffen sind, dass 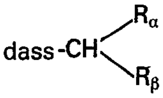 die bereits angegebene Bedeutung von $R_1$ mit Ausnahme eines Wasserstoffatoms und des Methylrestes besitzt, in Gegenwart eines Reduktionsmittels umsetzt, um ein Produkt der Formel $I_B$

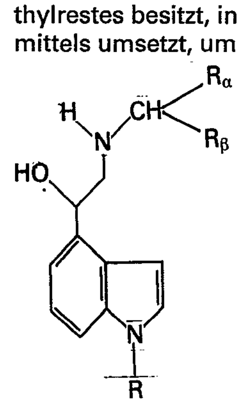

($I_B$)

oder $I'_B$

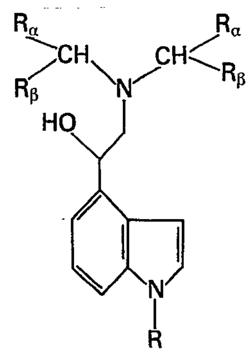

($I'_B$)

zu erhalten, worin R, $R_\alpha$ und $R_\beta$ die angegebene Bedeutung besitzen, das Produkt der Formel $I_B$ oder $I'_B$ isoliert und gewünschtenfalls in ein Salz überführt, oder das Produkt der Formel $I_B$ der Einwirkung eines Halogenids der Formel V

$$X\text{–}R'_2 \qquad (V)$$

unterzieht, worin X ein Chlor-, Brom- oder Jodatom bedeutet und $R'_2$ die für $R_2$ angegebene Bedeutung mit Ausnahme eines Wasserstoffatoms besitzt, um ein Produkt der Formel $I_C$

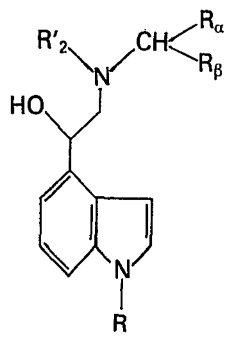

($I_C$)

zu erhalten, worin R, $R_\alpha$, $R_\beta$ und $R'_2$ die angegebene Bedeutung besitzen, das man isoliert und gewünschtenfalls in ein Salz überführt,
oder das Produkt der Formel $I_A$ mit einem Carbonylderivat der Formel $IV_a$

$Hal-\overset{O}{\overset{\|}{C}}-R'_1$ ($IV_a$)

umsetzt, worin Hal ein Chlor-, Brom- oder Jodatom bedeutet, und $-\overset{O}{\overset{\|}{C}}-R'_1$ so beschaffen ist, dass $-CH_2-R'_1$ die für $R_1$ angegebene Bedeutung mit Ausnahme eines Wasserstoffatoms und des Methylrestes besitzt, danach das erhaltene Derivat der Einwirkung eines Reduktionsmittels unterzieht, um ein Produkt der Formel $I_D$

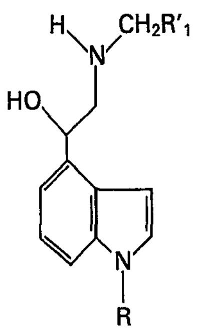

($I_D$)

zu erhalten, worin R und $R_1$ die angegebene Bedeutung besitzen, das Produkt der Formel $I_D$ entweder isoliert und gewünschtenfalls in ein Salz überführt oder der Einwirkung eines Halogenids der Formel V

$X-R'_2$ (V)

unterzieht, worin X und $R'_2$ die angegebene Bedeutung besitzen, um ein Produkt der Formel $I_E$

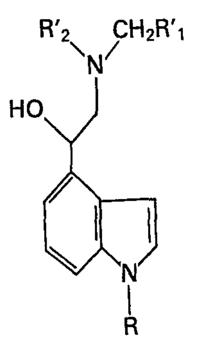

($I_E$)

zu erhalten, worin R, $R'_1$ und $R'_2$ die angegebene Bedeutung besitzen, das man isoliert und gewünschtenfalls in ein Salz überführt,
oder das Produkt der Formel $I_A$ einer Methylierung unterzieht, um ein Produkt der Formel $I_F$

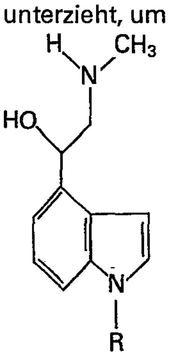

($I_F$)

zu erhalten, worin R die angegebene Bedeutung besitzt, das man entweder isoliert und gewünschtenfalls in ein Salz überführt oder mit einem Halogenid der Formel V, wie vorstehend definiert, umsetzt, um ein Produkt der Formel $I_G$

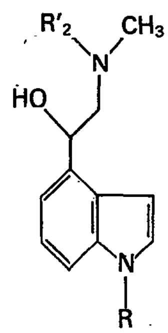

($I_G$)

zu erhalten, worin R und $R'_2$ die angegebene Bedeutung besitzen, das man isoliert und gewünschtenfalls in ein Salz überführt, und gewünschtenfalls die Produkte der Formeln $I_A$, $I_B$, $I'_B$, $I_C$, $I_D$, $I_E$, $I_F$ und $I_G$ der Einwirkung eines Reduktionsmittels für den Indolkern unterzieht, um ein Produkt der Formel I'

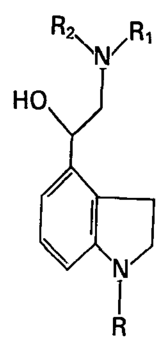

(I')

zu erhalten, worin R, $R_1$ und $R_2$ die angegebene Bedeutung besitzen, das man isoliert und gewünschtenfalls in ein Salz überführt.

6. Abänderung des Herstellungsverfahrens gemäss Anspruch 5, dadurch gekennzeichnet, dass man 4-Chlorindol mit einem Halogenid der Formel $V_a$

Hal–R' ($V_a$)

worin Hal ein Chlor-, Brom- oder Jodatom bedeutet und R' die für R angegebene Bedeutung mit

Ausnahme von Wasserstoff besitzt, umsetzt, um ein Produkt der Formel VI

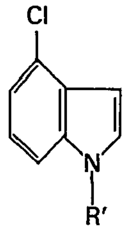

(VI)

zu erhalten, worin R' die angegebene Bedeutung besitzt, das man in die entsprechende Magnesiumverbindung überführt, die man mit einem Acetylierungsmittel umsetzt, um ein Produkt der Formel VII

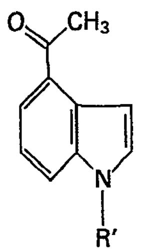

(VII)

zu erhalten, worin R' die angegebene Bedeutung besitzt, dessen Oxim man in α-Stellung des Ketons herstellt, danach das erhaltene Produkt reduziert, um ein Produkt der Formel $I'_A$

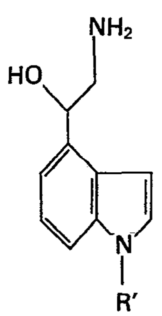

($I'_A$)

zu erhalten, worin R' die angegebene Bedeutung besitzt, das man entweder isoliert und gewünschtenfalls in ein Salz überführt, oder das Verfahren, wie in Anspruch 5 für das Produkt der Formel $I_A$ angegeben, fortsetzt.

7. Abänderung des Verfahrens gemäss Anspruch 5 zur Herstellung der Produkte der Formel I, worin $R_1$ einen linearen oder verzweigten Alkylrest mit 2 bis 8 Kohlenstoffatomen bedeutet, der durch einen oder mehrere Hydroxyreste, von denen sich zumindest ein Hydroxyrest an dem Kohlenstoffatom in 2-Stellung befindet, und ausserdem gegebenenfalls durch ein oder mehrere Halogenatome oder durch einen oder mehrere Aryl- oder Aryloxyreste, die gegebenenfalls an dem Arylring substituiert sind, substituiert ist, und $R_2$ ein Wasserstoffatom oder einen Alkylrest mit 1 bis 8 Kohlenstoffatomen bedeutet, der gegebenenfalls durch einen Arylrest substituiert ist, dadurch gekennzeichnet, dass man ein Produkt der Formel $I_H$

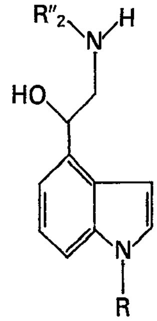

($I_H$)

worin R wie vorstehend definiert ist und $R''_2$ einen Alkylrest mit 1 bis 8 Kohlenstoffatomen bedeutet, der gegebenenfalls durch einen Arylrest substituiert ist, entsprechend einem Produkt der Formel $I_D$, wie vorstehend definiert, worin $-CH_2-R'_1$ die vorstehend angegebene Bedeutung von $R''_2$ besitzt, mit einem Reagens der Formel VIII

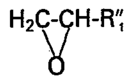

(VIII)

worin $R''_1$ derart ist, dass 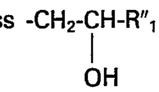 die vorstehend angegebene Bedeutung von $R_1$ besitzt, behandelt, um ein Produkt der Formel $I_I$

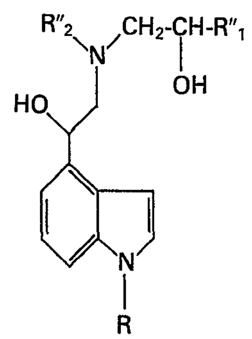

($I_I$)

zu erhalten, das man gegebenenfalls der Einwirkung eines Reduktionsmittels unterzieht, um eine Verbindung der Formel $I_J$

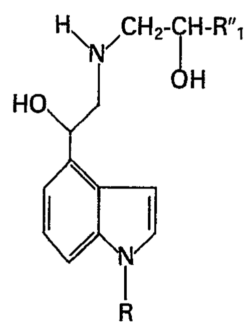

($I_J$)

zu erhalten, die man

entweder isoliert und gewünschtenfalls in ein Salz überführt oder wie die Produkte der Formel $I_B$, $I_D$ oder $I_F$ der Einwirkung eines Halogenids der Formel V, wie vorstehend definiert, unterzieht, um ein Produkt der Formel $I_K$

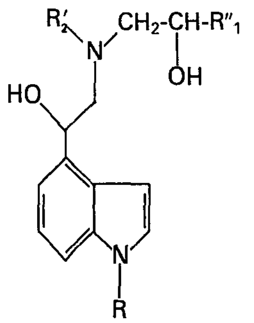

($I_K$)

zu erhalten, das man isoliert und gewünschtenfalls in ein Salz überführt und gewünschtenfalls die Produkte der Formel $I_I$, $I_J$ oder $I_K$ der Einwirkung eines Reduktionsmittels für den Indolkern unterzieht, um das entsprechende reduzierte Produkt zu erhalten, das man isoliert und gewünschtenfalls in ein Salz überführt.

8. Arzneimittel, dadurch gekennzeichnet, dass sie aus den neuen Aminomethyl-1H-indol-4-methanol-Derivaten der Formel I gemäss Anspruch 1 sowie aus deren Additionssalzen mit pharmazeutisch verträglichen Säuren bestehen.

9. Arzneimittel, dadurch gekennzeichnet, dass sie aus den neuen Aminomethyl-1H-indol-4-methanol-Derivaten gemäss einem der Ansprüche 2 und 3 sowie aus deren Additionssalzen mit pharmazeutisch verträglichen Säuren bestehen.

10. Arzneimittel, dadurch gekennzeichnet, dass sie aus den Aminomethyl-1H-indol-4-methanol-Derivaten gemäss Anspruch 4 sowie aus deren Additionssalzen mit pharmazeutisch verträglichen Säuren bestehen.

11. Pharmazeutische Zusammensetzungen, dadurch gekennzeichnet, dass sie als Wirkstoff zumindest eines der Arzneimittel gemäss einem der Ansprüche 8, 9 oder 10 enthalten.

12. Als neue, industrielle Produkte die Produkte der Formel III

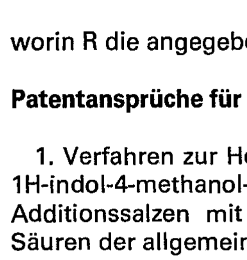

(III)

worin R die angegebene Bedeutung besitzt.

**Patentansprüche für den Vertragsstaat: AT**

1. Verfahren zur Herstellung der Aminomethyl-1H-indol-4-methanol-Derivate sowie von deren Additionssalzen mit Mineral- oder organischen Säuren der allgemeinen Formel I

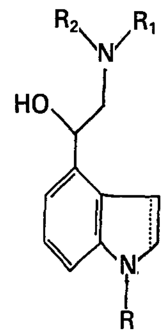

(I)

worin R ein Wasserstoffatom oder einen Alkylrest mit 1 bis 6 Kohlenstoffatomen oder einen Phenylalkylrest mit 7 bis 10 Kohlenstoffatomen bedeutet und $R_1$ und $R_2$ gleich oder verschieden sind und ein Wasserstoffatom, einen linearen oder verzweigten Alkylrest mit 1 bis 8 Kohlenstoffatomen, der gegebenenfalls mit Ausnahme des Falls des Methylrestes durch ein oder mehrere Halogenatome oder durch ein oder mehrere Hydroxy-, Phenyl- oder Phenoxyreste, die ihrerseits gegebenenfalls durch ein oder mehrere Halogenatome oder durch einen oder mehrere Hydroxy-, Methyl-, Methoxy- oder Acetamidoreste substituiert sein können, substituiert ist, bedeuten, $R_1$ und $R_2$ auch einen Cycloalkylrest mit 3 bis 7 Kohlenstoffatomen, einen Cycloalkylalkylrest mit 4 bis 7 Kohlenstoffatomen oder einen Allyl- oder Propargylrest bedeuten können und die gestrichelte Linie die etwaige Anwesenheit einer Kohlenstoff-Kohlenstoff-Bindung anzeigt, dadurch gekennzeichnet, dass man ein Produkt der Formel II

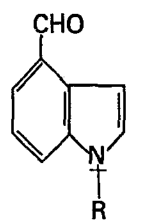

(II)

worin R die angegebene Bedeutung besitzt, mit Trimethylsilylcyanid umsetzt, um ein Produkt der Formel III

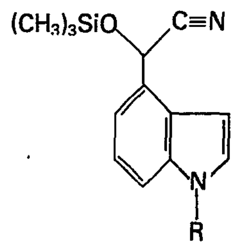

(III)

zu erhalten, worin R die angegebene Bedeutung besitzt, das man reduziert, um ein Produkt der Formel $I_A$

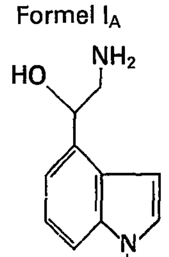

($I_A$)

zu erhalten, worin R die angegebene Bedeutung besitzt, das man entweder isoliert und gewünschtenfalls in ein Salz überführt oder das Produkt der Formel $I_A$ mit einem Carbonylderivat der Formel IV

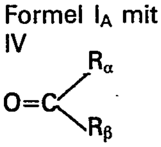

(IV)

worin $R_\alpha$ und $R_\beta$ so beschaffen sind, dass 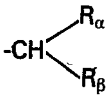 die bereits angegebene Bedeutung von $R_1$ mit Ausnahme eines Wasserstoffatoms und des Methylrestes besitzt, in Gegenwart eines Reduktionsmittels umsetzt, um ein Produkt der Formel $I_B$

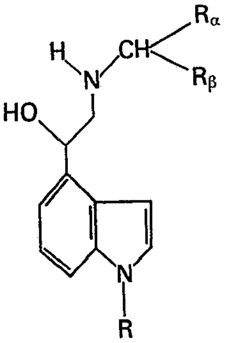

($I_B$)

oder $I'_B$

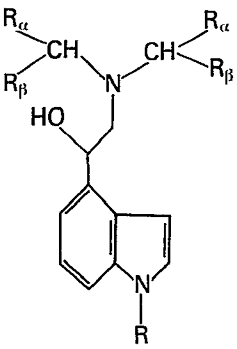

($I'_B$)

zu erhalten, worin R, $R_\alpha$ und $R_\beta$ die angegebene Bedeutung besitzen, das Produkt der Formel $I_B$ oder $I'_B$ isoliert und gewünschtenfalls in ein Salz überführt, oder das Produkt der Formel $I_B$ der Einwirkung eines Halogenids der Formel V

$X–R'_2$ (V)

unterzieht, worin X ein Chlor-, Brom- oder Jodatom bedeutet und $R'_2$ die für $R_2$ angegebene Bedeutung mit Ausnahme eines Wasserstoffatoms besitzt, um ein Produkt der Formel $I_C$

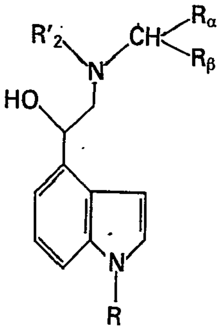

($I_C$)

zu erhalten, worin R, $R_\alpha$, $R_\beta$ und $R'_2$ die angegebene Bedeutung besitzen, das man isoliert und gewünschtenfalls in ein Salz überführt,
oder das Produkt der Formel $I_A$ mit einem Carbonylderivat der Formel $IV_a$

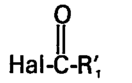

($IV_a$)

umsetzt, worin Hal ein Chlor-, Brom- oder Jodatom bedeutet, und 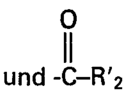 so beschaffen ist, dass $-CH_2-R'_1$ die für $R_1$ angegebene Bedeutung mit Ausnahme eines Wasserstoffatoms und des Methylrestes besitzt, danach das erhaltene Derivat der Einwirkung eines Reduktionsmittels unterzieht, um ein Produkt der Formel $I_D$

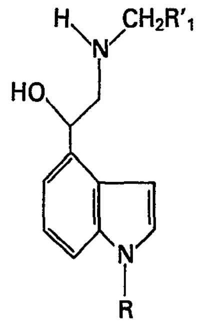

($I_D$)

zu erhalten, worin R und $R'_1$ die angegebene Bedeutung besitzen, das Produkt der Formel $I_D$ entweder isoliert und gewünschtenfalls in ein Salz überführt oder der Einwirkung eines Halogenids der Formel V

$X–R'_2$ (V)

unterzieht, worin X und $R'_2$ die angegebene Bedeutung besitzen, um ein Produkt der Formel $I_E$

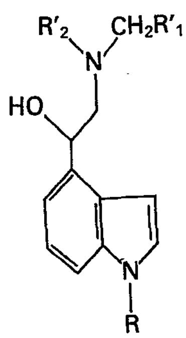

($I_E$)

zu erhalten, worin R, $R'_1$ und $R'_2$ die angegebene Bedeutung besitzen, das man isoliert und gewünschtenfalls in ein Salz überführt,
oder das Produkt der Formel $I_A$ einer Methylierung unterzieht, um ein Produkt der Formel $I_F$

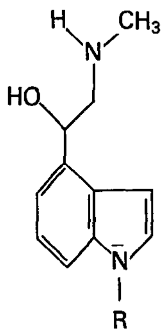

($I_F$)

zu erhalten, worin R die angegebene Bedeutung besitzt, das man entweder isoliert und gewünschtenfalls in ein Salz überführt oder mit einem Halogenid der Formel V, wie vorstehend definiert, umsetzt, um ein Produkt der Formel $I_G$

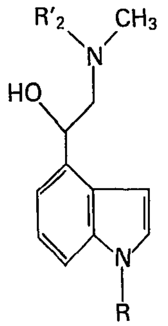

($I_G$)

zu erhalten, worin R und $R'_2$ die angegebene Bedeutung besitzen, das man isoliert und gewünschtenfalls in ein Salz überführt und gewünschtenfalls die Produkte der Formeln $I_A$, $I_B$, $I'_B$, $I_C$, $I_D$, $I_E$, $I_F$, und $I_G$ der Einwirkung eines Reduktionsmittels für den Indolkern unterzieht, um ein Produkt der Formel I'

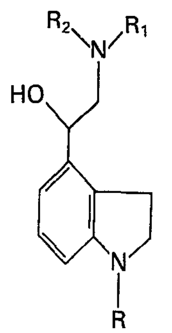

(I')

zu erhalten, worin R, $R_1$ und $R_2$ die angegebene Bedeutung besitzen, das man isoliert und gewünschtenfalls in ein Salz überführt.

2. Verfahren gemäss Anspruch 1, dadurch gekennzeichnet, dass man ein Produkt der Formel II

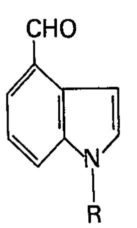

(II)

worin R die angegebene Bedeutung besitzt, mit Trimethylsilylcyanid umsetzt, um ein Produkt der Formel III

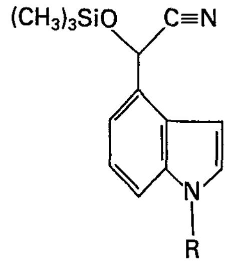

(III)

zu erhalten, worin R die angegebene Bedeutung besitzt, das man reduziert, um ein Produkt der Formel $I_A$

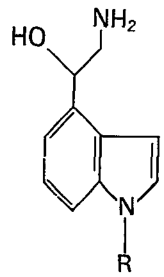

($I_A$)

zu erhalten, worin R die angegebene Bedeutung besitzt, das man entweder isoliert und gewünschtenfalls in ein Salz überführt oder das Produkt der Formel $I_A$ mit einem Carbonylderivat der Formel IV

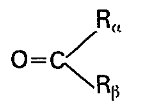

(IV)

worin $R_\alpha$ und $R_\beta$ derart sind, dass 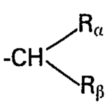

die für $R_1$ angegebene Bedeutung mit Ausnahme eines Wasserstoffatoms und des Methylrestes besitzt, in Gegenwart eines Reduktionsmittels umsetzt, um ein Produkt der Formel $I_B$

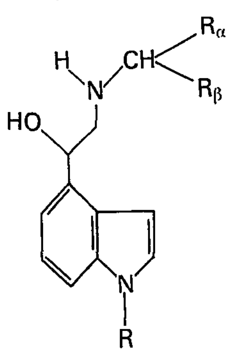

($I_B$)

zu erhalten, worin R, $R_\alpha$ und $R_\beta$ die angegebene Bedeutung besitzen, das Produkt der Formel $I_B$ entweder isoliert und gewünschtenfalls in ein Salz überführt oder der Einwirkung eines Halogenids der Formel V

$X–R'_2$ (V)

unterzieht, worin X ein Chlor-, Brom- oder Jodatom bedeutet und $R'_2$ die für $R_2$ angegebene

Bedeutung mit Ausnahme von Wasserstoff besitzt, um ein Produkt der Formel $I_C$

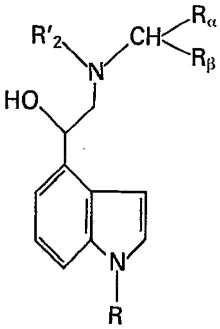

($I_C$)

zu erhalten, worin R, $R_\alpha$, $R_\beta$ und $R'_2$ die angegebene Bedeutung besitzen, das man isoliert und gewünschtenfalls in ein Salz überführt,
oder das Produkt der Formel $I_A$ einer Methylierung unterzieht, um ein Produkt der Formel $I_F$

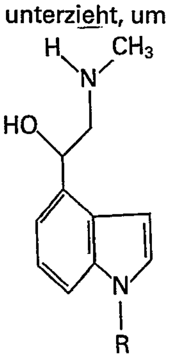

($I_F$)

zu erhalten, worin R die angegebene Bedeutung besitzt, das man entweder isoliert und gewünschtenfalls in ein Salz überführt oder mit einem Halogenid der Formel V, wie vorstehend definiert, umsetzt, um ein Produkt der Formel $I_G$

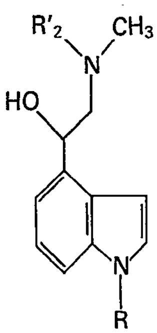

($I_G$)

zu erhalten, worin R und $R'_2$ die angegebene Bedeutung besitzen, das man isoliert und gewünschtenfalls in ein Salz überführt und gewünschtenfalls die Produkte der Formeln $I_A$, $I_B$, $I_C$, $I_F$ und $I_G$ der Einwirkung eines Reduktionsmittels für den Indolkern unterzieht, um ein Produkt der Formel I'

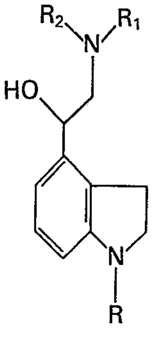

(I')

zu erhalten, worin R, $R_1$ und $R_2$ die angegebene Bedeutung besitzen, das man isoliert und gewünschtenfalls in ein Salz überführt.

3. Abänderung des Herstellungsverfahrens gemäss Anspruch 1, dadurch gekennzeichnet, dass man 4-Chlorindol mit einem Halogenid der Formel $V_a$

Hal–R' ($V_a$)

umsetzt, worin Hal ein Chlor-, Brom- oder Jodatom bedeutet und R' die für R angegebene Bedeutung mit Ausnahme von Wasserstoff besitzt, um ein Produkt der Formel VI

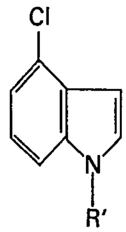

(VI)

zu erhalten, worin R' die angegebene Bedeutung besitzt, das man in die entsprechende Magnesiumverbindung überführt, die man mit einem Acetylierungsmittel umsetzt, um ein Produkt der Formel VII

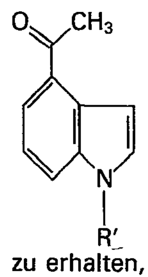

(VII)

zu erhalten, worin R' die angegebene Bedeutung besitzt, dessen Oxim man in α-Stellung des Ketons herstellt, danach das erhaltene Produkt reduziert, um ein Produkt der Formel $I'_A$

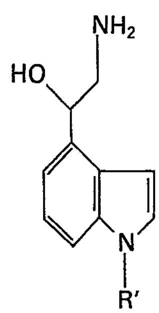

($I'_A$)

zu erhalten, worin R' die angegebene Bedeutung besitzt, das man entweder isoliert und gewünschtenfalls in ein Salz überführt oder das Verfahren fortsetzt, wie es in Anspruch 1 für das Produkt der Formel $I_A$ angegeben ist.

4. Abänderung des Verfahrens gemäss Anspruch 1 zur Herstellung der Produkte der Formel I, worin $R_1$ einen linearen oder verzweigten Alkylrest mit 2 bis 8 Kohlenstoffatomen bedeutet, der durch einen oder mehrere Hydroxyreste, von denen sich zumindest ein Hydroxyrest an dem Kohlenstoffatom in 2-Stellung befindet, und ausserdem gegebenenfalls durch ein oder mehrere Halogenatome oder durch einen oder mehrere Aryl- oder Aryloxyreste substituiert ist, die gegebenenfalls an dem Arylrest substituiert sind, und $R_2$ ein Wasserstoffatom oder einen Alkylrest mit 1 bis 8 Kohlenstoffatomen bedeutet, der gegebenenfalls durch einen Arylrest substituiert ist, dadurch gekennzeichnet, dass man ein Produkt der Formel $I_H$

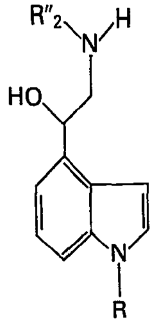

$(I_H)$

worin R wie vorstehend definiert ist und $R''_2$ einen Alkylrest mit 1 bis 8 Kohlenstoffatomen bedeutet, der gegebenenfalls durch einen Arylrest substituiert ist, entsprechend einem Produkt der Formel $I_D$, wie vorstehend definiert, worin $CH_2$-$R'_1$ die für $R''_2$ angegebene Bedeutung besitzt, mit einem Reagens der Formel VIII

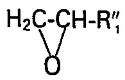

(VIII)

worin $R''_1$ derart ist, dass 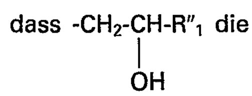 die vorstehend angegebene Bedeutung von $R_1$ besitzt, behandelt, um ein Produkt der Formel $I_I$

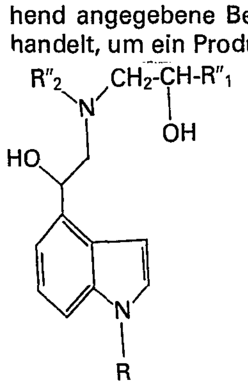

$(I_I)$

zu erhalten, das man gegebenenfalls der Einwirkung eines Reduktionsmittels unterzieht, um eine Verbindung der Formel $I_J$

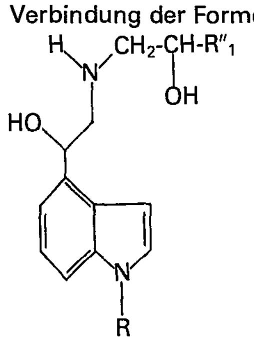

$(I_J)$

zu erhalten, die man entweder isoliert und gewünschtenfalls in ein Salz überführt oder wie die Produkte der Formel $I_B$, $I_D$ oder $I_F$ der Einwirkung eines Halogenids der Formel V, wie vorstehend definiert, unterzieht, um ein Produkt der Formel $I_K$

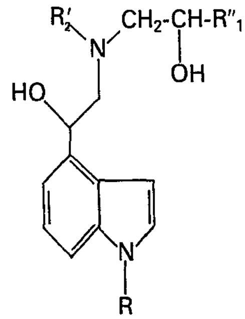

$(I_K)$

zu erhalten, das man isoliert und gewünschtenfalls in ein Salz überführt und gewünschtenfalls die Produkte der Formel $I_I$, $I_J$ oder $I_K$ der Einwirkung eines Reduktionsmittels für den Indolkern unterzieht, um das entsprechende reduzierte Produkt zu erhalten, das man isoliert und gewünschtenfalls in ein Salz überführt.

5. Verfahren gemäss einem der Ansprüche 1 bis 4, dadurch gekennzeichnet, dass man die Produkte der Formel I, wie in Anspruch 1 definiert, worin R ein Wasserstoffatom, einen Alkylrest mit 1 bis 3 Kohlenstoffatomen oder einen Benzylrest bedeutet, $R_1$ und $R_2$ ein Wasserstoffatom oder einen linearen oder verzweigten Alkylrest mit 1 bis 8 Kohlenstoffatomen, der gegebenenfalls substituiert ist, bedeuten sowie deren Additionssalze mit Mineral- oder organischen Säuren herstellt.

6. Verfahren gemäss einem der Ansprüche 1 bis 3, dadurch gekennzeichnet, dass man die Produkte der Formel I, wie in Anspruch 1 definiert, worin R ein Wasserstoffatom bedeutet, $R_1$ und $R_2$ ein Wasserstoffatom oder einen linearen oder verzweigten Alkylrest mit 1 bis 8 Kohlenstoffatomen, der gegebenenfalls durch einen Phenyl- oder Phenoxyrest substituiert ist, die ihrerseits gegebenenfalls durch ein Halogenatom substituiert sind, bedeuten und die gestrichelte Linie eine Kohlenstoff-Kohlenstoff-Bindung anzeigt sowie deren Additionssalze mit Mineral- oder organischen Säuren herstellt.

7. Verfahren gemäss einem der Ansprüche 1 bis 3, dadurch gekennzeichnet, dass man irgendeines der Derivate mit den folgenden Bezeichnungen sowie deren Additionssalze mit Mineral- oder organischen Säuren herstellt:
das α-(Aminomethyl)-1H-indol-4-methanol,
das α-[[(1-Methylethyl)-amino]-methyl]-1H-indol-4-methanol,
das α-(Methylamino)-methyl-1H-indol-4-methanol,
das α-[[(1-Propyl)-amino]-methyl]-1H-indol-4-methanol.

**Claims for the contracting states**
**BE, CH, DE, FR, GB, IT, LI, LU, NL, SE**

1. Derivates of aminomethyl 1H-indol-4-methanol as well as their salts of addition with mineral or organic acids, characterized in that they answer to the general formula I:

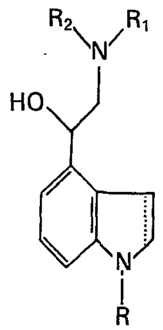

(I)

in which R represents a hydrogen atom, or an alkyl radical containing from 1 to 6 carbon atoms or a phenyl alkyl radical containing from 7 to 10 carbon atoms and $R_1$ and $R_2$, being identical or different, each represents a hydrogen atom, a linear or branched alkyl radical containing from 1 to 8 carbon atoms, possibly substituted, except in the case of the methyl radical, by one or more halogen atoms or by one or more hydroxy, phenyl or phenoxy radicals themselves possibly substituted by one or more halogen atoms or by one or more hydroxy, methyl, methoxy or acetamido radicals, $R_1$ and $R_2$ can also represent a cycloalkyl radical containing from 3 to 7 carbon atoms, a cycloalkylalkyl radical containing from 4 to 7 carbon atoms or an allyl or propargyl radical and the dotted line represents the possible presence of a carbon-carbon bond.

2. Derivatives of aminomethyl-1H-indol-4-methanol, as defined by formula I of claim 1, as well as their salts of addition with mineral or organic acids, characterized in that R represents a hydrogen atom, an alkyl radical containing from 1 to 3 carbon atoms or a benzyl radical, $R_1$ and $R_2$ each represent a hydrogen atom or a linear or branched alkyl radical, containing from 1 to 8 carbon atoms possibly substituted.

3. Derivatives of aminomethyl 1H-indol-4-methanol according to claim 2, as well as their salts of addition with mineral or organic acids, characterized in that R represents a hydrogen atom, $R_1$ and $R_2$ each represents a hydrogen atom or a linear or branched alkyl radical containing from 1 to 8 carbon atoms, possibly substituted by a phenyl or phenoxy radical, themselves possibly substituted by a halogen atom and the dotted line represents a carbon-carbon bond.

4. Any one of the derivatives according to claim 1, the names of which follow as well as their salts of addition with mineral or organic acids:

α-(aminomethyl)-1H-indol-4-methanol,

α-[[(1-methylethyl)amino]methyl]-1H-indol-4-methanol,

α-(methylamino)methyl-1H-indol-4-methanol,

α-[[(1-propyl)amino]methyl]-1H-indol-4-methanol.

5. Preparation process of derivatives answering to formula I of claim 1 as well as their salts of addition with mineral or organic acids, characterized in that a product with the formula II:

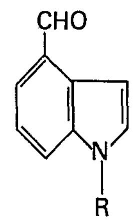

(II)

in which R has the significance already indicated, is made to react with trimethyl silyl cyanide, so as to obtain a product with the formula III:

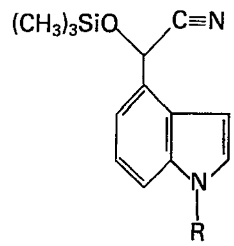

(III)

in which R has the significance already indicated, which is reduced, so as to obtain a product with the formula $I_A$:

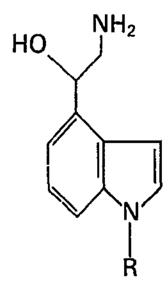

($I_A$)

in which R has the significance already indicated which:

either is isolated, and if desired, is salified,

or the said product with the formula $I_A$ is made to react with a carbonyl derivative with the formula IV:

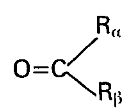

(IV)

in which Rα and Rβ are such that: 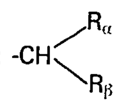 has the significance of $R_1$ already indicated with the exception of a hydrogen atom and a methyl radical, in the presence of a reducing agent, so as to obtain a product with the formula $I_B$:

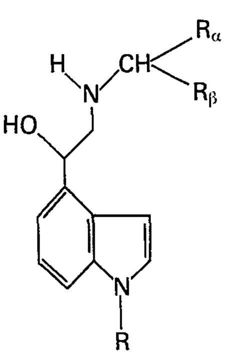

($I_B$)

or $I'_B$

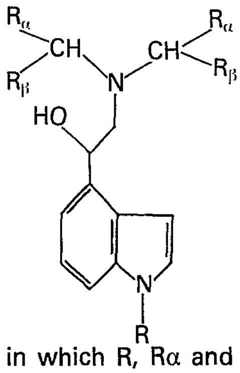

($I'_B$)

in which R, Rα and Rβ have the significance already indicated, which product with the formula $I_B$ or $I'_B$ is isolated and if desired, salified or which product with the formula $I_B$ is submitted to the action of a halogenide with the formula V:

$X–R'_2$ (V)

in which X represents a chlorine, bromine or iodine atom, and $R'_2$ has the significance of $R_2$ already indicated with the exception of hydrogen, so as to obtain a product with the formula $I_C$:

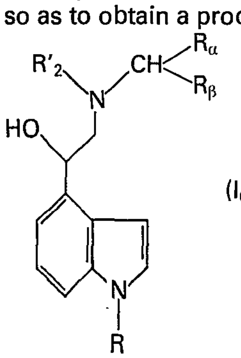

($I_C$)

in which R, Rα, Rβ and $R'_2$ have the significance already indicated which is isolated and if desired, salified, or the said product with the formula $I_A$ is made to react with a carbonyl derivative with the formula $IV_a$:

($IV_a$)

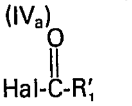

. ($IV_a$)

in which Hal represents a chlorine, bromine or iodine atom, and $-\underset{\displaystyle O}{\underset{||}{C}}-R'_1$ is such that $-CH_2-R'_1$ has the significance of $R_1$ already indicated, with the exception of a hydrogen atom and of the methyl radical, then the derivative obtained is submitted to the action of a reducing agent, so as to obtain a product with the formula $I_D$:

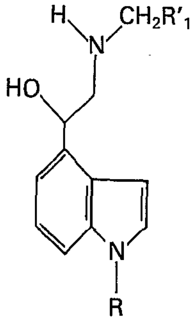

($I_D$)

in which R and $R'_1$ have the significance already indicated, which product with the formula $I_D$ is either isolated and if desired, salified, or is submitted to the action of a halogenide with the formula V:

$X–R'_2$ (V)

in which X and $R'_2$ have the significance already indicated, so as to obtain a product with the formula $I_E$:

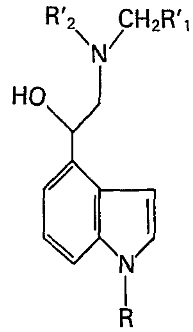

($I_E$)

in which R, $R'_1$ and $R'_2$ have the significance already indicated, which is isolated, and if desired, salified

or the said product with the formula $I_A$ is submitted to methylation, so as to obtain a product with the formula $I_F$:

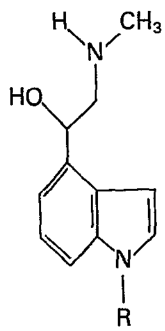

($I_F$)

in which R has the significance already indicated, which is either isolated, and if desired, salified, or is made to react with a halogenide with the formula V defined above, so as to obtain a product with the formula $I_G$:

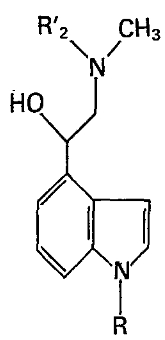

($I_G$)

in which R and $R'_2$ have the significance already indicated, which is isolated and if desired, salified, and if desired, the said products with the formulae $I_A$, $I_B$, $I'_B$, $I_C$, $I_D$, $I_E$, $I_F$ and $I_G$, are submitted to the action of a reducing agent of the indole nucleus, so as to obtain a product with the formula I':

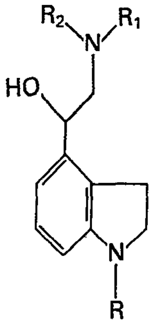

(I')

in which R, $R_1$ and $R_2$ have the significance already indicated, which is isolated, and if desired, salified.

6. Variant of the preparation process according to claim 5, characterized in that 4-chloro-indole is made to react with a halogenide with the formula $V_a$:

Hal–R' ($V_a$)

in which Hal represents a chlorine, bromine or iodine atom and R' has the significance of R already indicated, with the exception of hydrogen, so as to obtain a product with the formula VI:

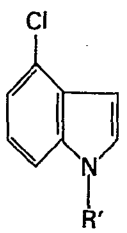

(VI)

in which R' has the significance already indicated, which is converted into the corresponding magnesian derivative, on which an acetylation agent is made to react, so as to obtain a product with the formula VII:

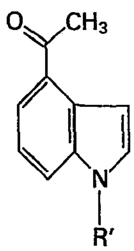

(VII)

in which R' has the significance already indicated, of which the oxime at α of the ketone is prepared, then the product obtained is reduced, so as to obtain a product with the formula $I'_A$:

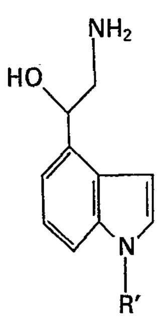

($I'_A$)

in which R' has the significance already indicated, which either is isolated and, if desired, salified, or the process as indicated in claim 5 for the product with the formula $I_A$ is followed.

7. Variant of the process according to claim 5, for the preparation of products with the formula I, in which $R_1$ represents a linear or branched alkyl radical containing from 2 to 8 carbon atoms, substituted by one or more hydroxy radicals of which at least one hydroxy is on the carbon at position 2 and, furthermore, possibly substituted by one or more halogen atoms or by one or more aryl or aryloxy radicals possibly substituted on the aryl nucleus and $R_2$ represents a hydrogen atom or an alkyl radical containing from 1 to 8 carbon atoms, possibly substituted by an aryl radical, which variant is characterized in that a product with the formula $I_H$:

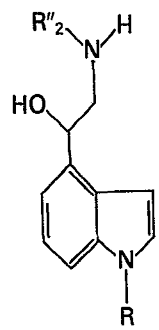

($I_H$)

in which R is defined as above and $R''_2$ represents an alkyl radical containing from 1 to 8 carbon atoms, possibly substituted by an aryl radical corresponding to a product with the formula $I_D$ as defined previously, in which $CH_2$-$R'_1$ has the significance of $R''_2$ indicated above, is treated with a reagent with the formula VIII:

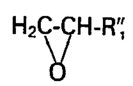

(VIII)

in which $R''_1$ is such that 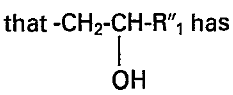 has the value of $R_1$ indicated above, so as to obtain a product with the formula $I_I$:

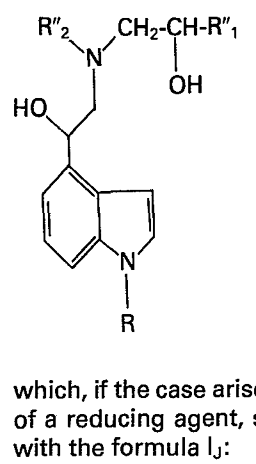

($I_I$)

which, if the case arises, is submitted to the action of a reducing agent, so as to obtain a compound with the formula $I_J$:

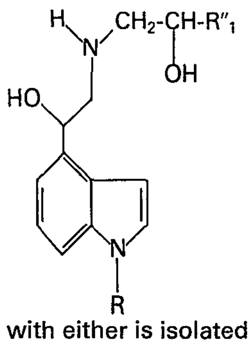

($I_J$)

with either is isolated and, if desired, salified, or is submitted, like the products with the formula $I_B$, $I_D$ or $I_F$, to the action of a halogenide with the formula V as defined previously, so as to obtain a product with the formula $I_K$:

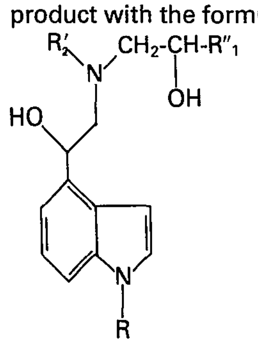

($I_K$)

which is isolated and, if desired, salified and, if desired, the products with the formula $I_I$, $I_J$ or $I_K$ are submitted to the action of a reducing agent of the indole nucleus, so as to obtain the corresponding reduced product which is isolated and, if desired, salified.

8. Medicaments, characterized in that they are composed of new derivatives of aminomethyl 1H-indol-4-methanol, as defined by the formula I of claim 1, as well as by their salts of addition with pharmaceutically acceptable acids.

9. Medicaments, characterized in that they are composed of new derivatives of aminomethyl 1H-indol-4-methanol, as defined in either of the claims 2 or 3 as well as by their salts of addition with pharmaceutically acceptable acids.

10. Medicaments, characterized in that they are composed of derivatives of aminomethyl 1H-indol-4-methanol as defined in claim 4 as well as by their salts of addition with pharmaceutically acceptable acids.

11. Pharmaceutical compositions, characterized in that they contain, as activer principle, at least one of the medicaments, as defined in one of the claims 8, 9 or 10.

12. As new industrial products, products with the formula III:

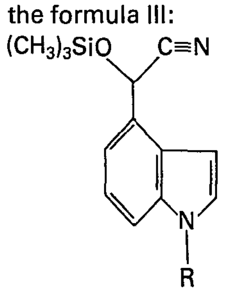

(III)

in which R has the significance already indicated.

**Claims for the contracting state AT**

1. Process for preparing derivatives of aminomethyl 1H-indol-4-methanol as well as their salts of addition with mineral or organic acids, answering to the general formula I:

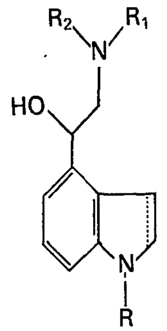

(I)

in which R represents a hydrogen atom, or an alkyl radical containing from 1 to 6 carbon atoms or a phenyl alkyl radical containing from 7 to 10 carbon atoms and $R_1$ and $R_2$, being identical or different, each represent a hydrogen atom, a linear or branched alkyl radical containing from 1 to 8 carbon atoms, possibly substituted, except in the case of the methyl radical, by one or more halogen atoms or by one or more hydroxy, phenyl or phenoxy radicals themselves possibly substituted by one or more halogen atoms or by one or more hydroxy, methyl, methoxy or acetamido radicals, $R_1$ and $R_2$ can also represent a cycloalkyl radical containing from 3 to 7 carbon atoms, a cycloalkylalkyl radical containing from 4 to 7 carbon atoms or an allyl or propargyl radical and the dotted line represents the possible presence of a carbon-carbon bond, characterized in that a product with the formula II:

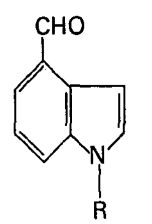

(II)

in which R has the significance already indicated, is made to react with trimethyl silyl cyanide, so as to obtain a product with the formula III:

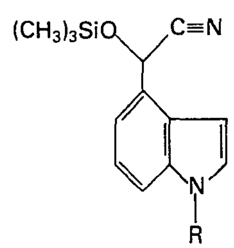

(III)

in which R has the significance already indicated, which is reduced, so as to obtain a product with the formula $I_A$:

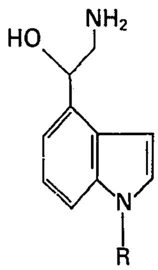

$(I_A)$

in which R has the significance already indicated which:
either is isolated, and if desired, is salified,
or the said product with the formula $I_A$ is made to react with a carbonyl derivative with the formula IV:

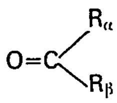

(IV)

in which Rα and Rβ are such that: 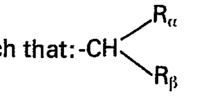

has the significance of $R_1$ already indicated with the exception of a hydrogen atom and a methyl radical, in the presence of a reducing agent, so as to obtain a product with the formula $I_B$:

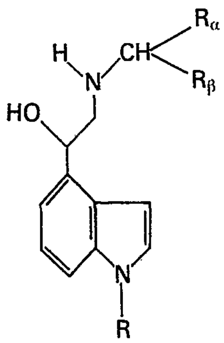

$(I_B)$

or $I'_B$

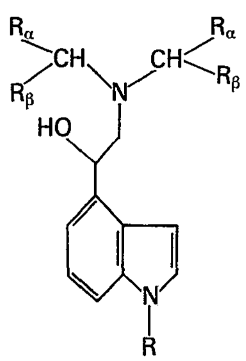

$(I'_B)$

in which R, Rα and Rβ have the significance already indicated, which product with the formula $I_B$ or $I'_B$ is isolated and if desired, salified or which product with the formula $I_B$ is submitted to the action of a halogenide with the formula V:

$X–R'_2$ (V)

in which X represents a chlorine, bromine or iodine atom, and $R'_2$ has the significance of $R_2$ already indicated with the exception of hydrogen, so as to obtain a product with the formula $I_C$:

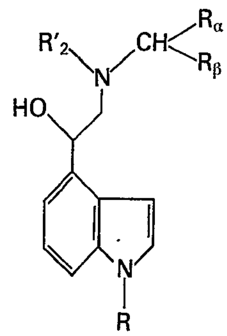

$(I_C)$

in which R, Rα, Rβ and $R'_2$ have the significance already indicated which is isolated and if desired, salified, or the said product with the formula $I_A$ is made to react with a carbonyl derivative with the formula $IV_a$:

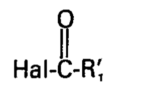

$(IV_a)$

in which Hal represents a chlorine, bromine or iodine atom, and 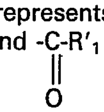 is such that $-CH_2-R'_1$ has the significance of $R_1$ already indicated, with the exception of a hydrogen atom and of the methyl radical, then the derivative obtained is submitted to the action of a reducing agent, so as to obtain a product with the formula $I_D$:

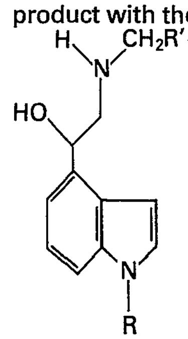

$(I_D)$

in which R and $R'_1$ have the significance already indicated, which product with the formula $I_D$ is either isolated and if desired, salified, or is submitted to the action of a halogenide with the formula V:

$X–R'_2$ (V)

in which X and $R'_2$ have the significance already indicated, so as to obtain a product with the formula $I_E$:

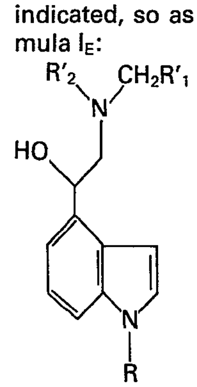

$(I_E)$

in which R, $R'_1$ and $R'_2$ have the significance already indicated, which is isolated, and if desired,

salified, or the said product with the formula $I_A$ is submitted to methylation, so as to obtain a product with the formula $I_F$:

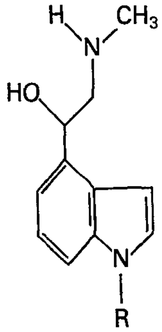

($I_F$)

in which R has the significance already indicated, which is either isolated, and if desired, salified, or is made to react with a halogenide with the formula V defined above, so as to obtain a product with the formula $I_G$:

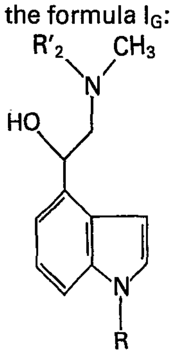

($I_G$)

in which R and $R'_2$ have the significance already indicated, which is isolated and if desired, salified, and if desired, the said products with the formulae $I_A$, $I_B$, $I'_B$, $I_C$, $I_D$, $I_E$, $I_F$ and $I_G$, are submitted to the action of a reducing agent of the indole nucleus, so as to obtain a product with the formula I':

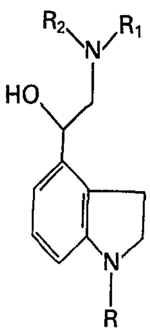

(I')

in which R, $R_1$ and $R_2$ have the significance already indicated, which is isolated, and if desired, salified.

2. Process according to claim 1, characterized in that a product with the formula II:

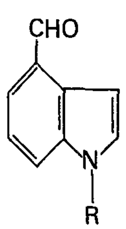

(II)

in which R has the significance already indicated, is made to react with trimethyl silyl cyanide, so as to obtain a product with the formula III:

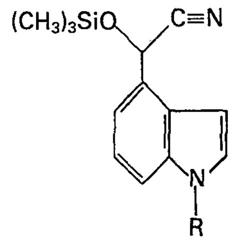

(III)

in which R has the significance already indicated, which is reduced, so as to obtain a product with the formula $I_A$:

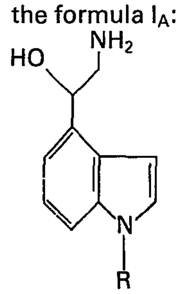

($I_A$)

in which R has the significance already indicated which:
either is isolated, and if desired, is salified,
or the said product with the formula $I_A$ is made to react with a carbonyl derivative with the formula IV:

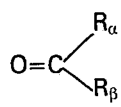

(IV)

in which Rα and Rβ are such that: 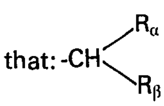

has the significance of $R_1$ already indicated with the exception of a hydrogen atom and a methyl radical, in the presence of a reducing agent, so as to obtain a product with the formula $I_B$:

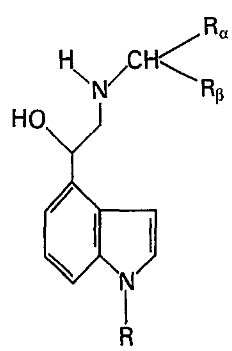

($I_B$)

in which R, Rα and Rβ have the significance already indicated, which product with the formula $I_B$ is either isolated and if desired, salified or submitted to the action of a halogenide with the formula V:

$$X\text{–}R'_2 \qquad (V)$$

in which X represents a chlorine, bromine or iodine atom, and $R'_2$ has the significance of $R_2$ already indicated with the exception of hydrogen, so as to obtain a product with the formula $I_C$:

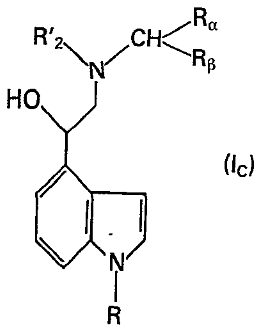

$(I_C)$

in which R, Rα, Rβ and R'$_2$ have the significance already indicated which is isolated and if desired, salified, or the said product with the formula $I_A$ is submitted to methylation, so as to obtain a product with the formula $I_F$:

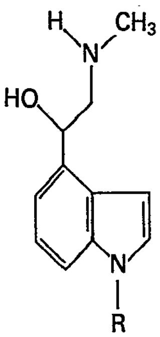

$(I_F)$

in which R has the significance already indicated, which is either isolated, and if desired, salified, or is made to react with a halogenide with the formula V defined above, so as to obtain a product with the formula $I_G$:

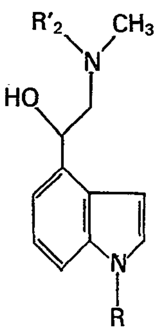

$(I_G)$

in which R and R'$_2$ have the significance already indicated, which is isolated and if desired, salified, and if desired, the said products with the formulae $I_A$, $I_B$, $I_C$, $I_F$ and $I_G$, are submitted to the action of a reducing agent of the indole nucleus, so as to obtain a product with the formula I':

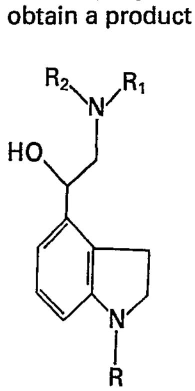

(I')

in which R, $R_1$ and $R_2$ have the significance already indicated, which is isolated, and if desired, salified.

3. Variant of the preparation process according to claim 1, characterized in that 4-chloro-indole is made to react with a halogenide with the formula $V_a$:

Hal–R' $(V_a)$

in which Hal represents a chlorine, bromine or iodine atom and R' has the significance of R already indicated, with the exception of hydrogen, so as to obtain a product with the formula VI:

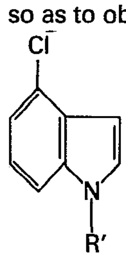

(VI)

in which R' has the significance already indicated, which is converted into the corresponding magnesian derivative, on which an acetylation agent is made to react, so as to obtain a product with the formula VII:

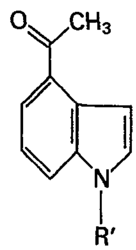

(VII)

in which R' has the significance already indicated, of which the oxime at α of the ketone, is prepared, then the product obtained is reduced, so as to obtain a product with the formula $I'_A$:

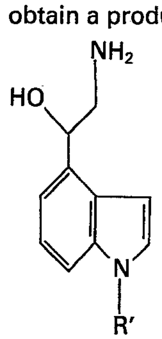

$(I'_A)$

in which R' has the significance already indicated, which either is isolated and, if desired, salified, or the process as indicated in claim 1 for the product with the formula $I_A$ is followed.

4. Variant of the process according to claim 4, for the preparation of products with the formula I, in which $R_1$ represents a linear or branched alkyl radical containing from 2 to 8 carbon atoms, substituted by one or more hydroxy radicals of which at least one hydroxy is on the carbon at position 2 and, furthermore, possibly substituted by one or more halogen atoms or by one or more aryl or aryloxy radicals possibly substituted on the aryl

nucleus and $R_2$ represents a hydrogen atom or an alkyl radical containing from 1 to 8 carbon atoms, possibly substituted by an aryl radical, which variant is characterized in that a product with the formula $I_H$:

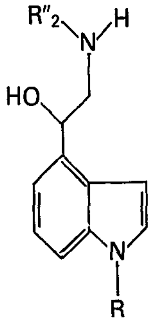

($I_H$)

in which R is defined as above and $R''_2$ represents an alkyl radical containing from 1 to 8 carbon atoms, possibly substituted by an aryl radical corresponding to a product with the formula $I_D$ as defined previously, in which $CH_2$-$R'_1$ has the significance of $R''_2$ indicated above, is treated with a reagent with the formula VIII:

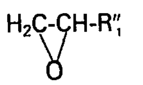

(VIII)

in which $R''_1$ is such that 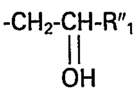 has the value of $R_1$ indicated above, so as to obtain a product with the formula $I_I$:

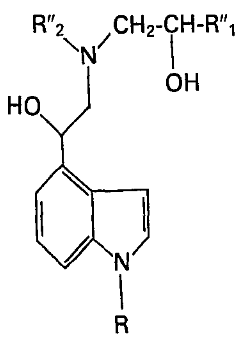

($I_I$)

which, if the case arises, is submitted to the action of a reducing agent, so as to obtain a compound with the formula $I_J$:

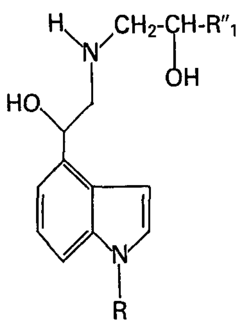

($I_J$)

which either is isolated and, if desired, salified, or is submitted, like the products with the formula $I_B$, $I_D$, $I_F$, to the action of a halogenide with the formula V as defined previously, so as to obtain a product with the formula $I_K$:

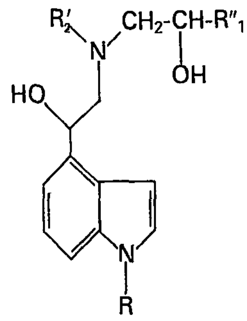

($I_K$)

which is isolated and, if desired, salified and, if desired, the products with the formula $I_I$, $I_J$ or $I_K$ are submitted to the action of a reducing agent of the indole nucleus, so as to obtain the corresponding reduced product which is isolated and, if desired, salified.

5. Process according to any one of the claims 1 to 4, characterized in that products with the formula I are prepared, as defined in claim 1, in which R represents a hydrogen atom, an alkyl radical containing from 1 to 3 carbon atoms or a benzyl radical, $R_1$ and $R_2$ each represent a hydrogen atom or a linear or branched alkyl radical, containing from 1 to 8 carbon atoms, possibly substituted, as well as their salts of addition with mineral or organic acids.

6. Process according to any one of the claims 1 to 3, characterized in that products with the formula I are prepared as defined in claim 1, in which R represents a hydrogen atom, $R_1$ and $R_2$ each represent a hydrogen atom or a linear or branched alkyl radical containing from 1 to 8 carbon atoms, possibly substituted by a phenyl or phenoxy radical, themselves possibly substituted by a halogen atom and the dotted line represents a carbon-carbon bond, as well as their salts of addition with mineral or organic acids.

7. Process according to any one of the claims 1 to 3, characterized in that any one of the derivatives are prepared, the names of which follow, as well as their salts of addition with mineral or organic acids:

α-(aminomethyl)-1H-indol-4-methanol,

α-[[(1-methylethyl)amino]methyl]-1H-indol-4-methanol,

α-(methylamino)methyl-1H-indol-4-methanol,

α-[[(1-propyl)amino]-methyl]-1H-indol-4-methanol.